(19)

Europäisches
Patentamt
European
Patent Office
Office européen
des brevets

(11) **EP 2 479 695 A2**

(12) **EUROPEAN PATENT APPLICATION**

(43) Date of publication:
**25.07.2012 Bulletin 2012/30**

(51) Int Cl.:
***G06F 19/00*** (2011.01)   ***G06F 19/16*** (2011.01)

(21) Application number: **12152328.6**

(22) Date of filing: **24.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.01.2011 JP 2011013284**
           **16.05.2011 JP 2011109754**

(71) Applicant: **Fujitsu Limited**
    **Kawasaki-shi, Kanagawa 211-8588 (JP)**

(72) Inventors:
 • **Sato, Hiroyuki**
   **Kawasaki-shi, Kanagawa 211-8588 (JP)**
 • **Matsuura, Azuma**
   **Kawasaki-shi, Kanagawa 211-8588 (JP)**

(74) Representative: **Fenlon, Christine Lesley**
    **Haseltine Lake LLP**
    **Lincoln House, 5th Floor**
    **300 High Holborn**
    **London WC1V 7JH (GB)**

(54) **Apparatus, method, and recording-medium for molecular structure construction and molecular structure design**

(57)    A molecular structure construction assisting apparatus includes a storage unit configured to store a data structure pertaining to a molecular structure, the data structure including at least a molecule data structure including an atom data structure pertaining to an atom, a bond axis data structure pertaining to a bond axis, and a molecule main data structure, an element data structure pertaining to an element of the atom, a display unit configured to display the molecular structure, an operation unit configured to instruct a target atom to be arranged in the molecular structure, a molecular structure design unit configured to derive a hybrid orbital of the target atom and a localized molecular orbital pertaining to a bond formed between the target atom and another atom of the molecular structure by using the data structure.

FIG.2

**Description**

FIELD

The embodiments discussed herein are related to an apparatus, a method, and a computer-readable recording medium for molecular structure construction and molecular structure design.

BACKGROUND

In recent state of the art research and development such as drug design development, there is a growing demand for molecular orbital calculation using computers. Because molecular structure data is to be input to a computer for performing the molecular orbital calculation (particularly, in the field of drug design where giant molecular structures such as protein are handled in large portions), a molecular structure construction assisting apparatus having a GUI (Graphical User Interface) that enables easy input of such molecular structures is used.

Typically, in a case of constructing a new molecular structure such as designing a molecule having a new characteristic, first, a new molecule is assembled by using a molecular structure construction assisting apparatus having the above-described GUI. Then, information such as the characteristics of the molecule is obtained by calculating the molecular orbital of the molecule by using a molecular orbital calculation program of a computer based on the structure of the molecule.

In performing the molecular orbital calculation, an initial electron density of the molecule is to be set. Many molecular orbital calculation programs use a method of setting the initial electron density matrix to a diagonal matrix and approximating the initial electron density matrix to the true electron density matrix by repeatedly performing calculation. In a case where the initial electron density (matrix) is significantly different from the true electron density (matrix), there is a problem in which a large amount of time is required due to repeating the calculation for a considerably large number of times. In some cases, the calculation may not even converge (particularly for giant molecular structures). Therefore, accurate setting of the initial electron density (matrix) becomes important.

In order to handle such problem, there is a method that uses LMO (Localized Molecular Orbitals) throughout an operation starting from setting the initial electron density and repeating calculation until the initial electron density is approximated to a true electron density. As one method, there is, for example, a MOZYME method (provided by Fujitsu Ltd.) which is performed by using LMO for constructing an initial electron density according to a Lewis structure and performing repeated calculation. As another method, there is a method where the LMO is not used for setting the initial electron density but is efficiently used in the repeated calculation by dividing a molecule into plural segments. The former method can configure an appropriate initial electron density and calculate electron density at high speed because the amount of calculation in each cycle of the repeated calculation can be omitted by narrowing the range of integral calculation. The latter method is also expected to perform high speed calculation in a case where the problem of large calculation amount pertaining to initial electron density or the problem of inconsistency upon combining molecular structures do not occur. There is also a method of diverting the electron density of a small molecule (which is to be a standard) to the setting of the initial electron density such that a more appropriate initial electron density matrix can be configured compared to using the diagonal matrix.

Patent document 1: Japanese Laid-open Patent Publication No. 7-168807

Patent document 2: Japanese Laid-open Patent Publication No. 2003-12567

Patent document 3: Japanese Laid-open Patent Publication No. 2001-319179

Non-patent document 1: P.W. Atkins, Hideaki Chihara, Nobuo Nakamura (joint translation), "Atkins Physical Chemistry", vol. 1 and 2, Tokyo Kagaku Dojin, February 2001

In using the conventional methods of setting the initial electron density (matrix) for high speed molecular orbital calculation, calculation is performed based on a molecule whose structure is already designed, and the result of the calculation is applied to a dedicated molecular orbital calculation program.

For example, in drug design development, there is a case where it is desired to understand, for example, the changes of physical property of respective molecules of proteins due to the difference of a given functional group. In this case, even if the structures of the molecules are substantially the same, it is necessary to calculate the molecular orbital of a molecule if the molecule has a part (e.g., functional group) that is slightly structurally different from the other molecules. With the conventional methods, even if the structures of the molecules are substantially the same, it is necessary to set the initial electron density matrix from scratch based on the entire molecular structure of the molecules, in order to obtain an appropriate initial electron density matrix having satisfactory convergence. As a result, a large amount of calculation is required. For example, with the above-described MOZYME method, even in a case of a molecule having a part that slightly structurally changed with respect to an original molecule, it is necessary to use all LMO on the molecule in the same manner as the original molecule. Therefore, there is a problem in which the amount of calculation for constructing the initial electron density increases as the number of molecules increases even in a case of molecules having similar molecular structures.

SUMMARY

According to an aspect of the invention, there is provided a molecular structure construction assisting apparatus including: a storage unit configured to store a data structure pertaining to a molecular structure, the data structure including at least a molecule data structure including an atom data structure pertaining to an atom, a bond axis data structure pertaining to a bond axis, and a molecule main data structure, an element data structure pertaining to an element of the atom; a display unit configured to display the molecular structure; an operation unit configured to instruct a target atom to be arranged in the molecular structure; a molecular structure design unit configured to derive a hybrid orbital of the target atom and a localized molecular orbital pertaining to a bond formed between the target atom and another atom of the molecular structure by using the data structure.

BRIEF DESCRIPTION OF DRAWINGS

Fig. 1 is a schematic diagram illustrating a hardware configuration of a computer of a molecular structure construction assisting apparatus for constructing a molecular structure according to an embodiment of the present invention;

Fig. 2 is a schematic diagram illustrating a functional configuration of a molecular structure construction assisting apparatus according to an embodiment of the present invention;

Fig. 3 is a schematic diagram illustrating the flow of LMO construction according to an embodiment of the present invention;

Figs. 4A-4C are schematic diagrams for describing a data structure according to an embodiment of the present invention;

Fig. 5 is a schematic diagram illustrating the flow of initializing a data structure according to an embodiment of the present invention;

Fig. 6 is a schematic diagram illustrating the flow of setting LMO according to an embodiment of the present invention (part 1);

Fig. 7 is a schematic diagram illustrating the flow of setting LMO according to an embodiment of the present invention (part 2);

Figs. 8A and 8B are schematic diagrams illustrating a molecular structure of ethylene;

Fig. 9 is a schematic diagram illustrating an electron density matrix of ethylene according to an embodiment of the present invention;

Fig. 10 is a schematic diagram for describing an interaction matrix with respect to elements of different atoms of a Fock matrix and ethylene according to an embodiment of the present invention;

Figs. 11A-11D are schematic diagrams for describing the flow and matrices of hybrid orbitals of ethylene according to an embodiment of the present invention; and

Figs. 12A-12C are schematic diagrams illustrating matrices of bond LMO of ethylene according to an embodiment of the present invention.

DESCRIPTION OF EMBODIMENTS

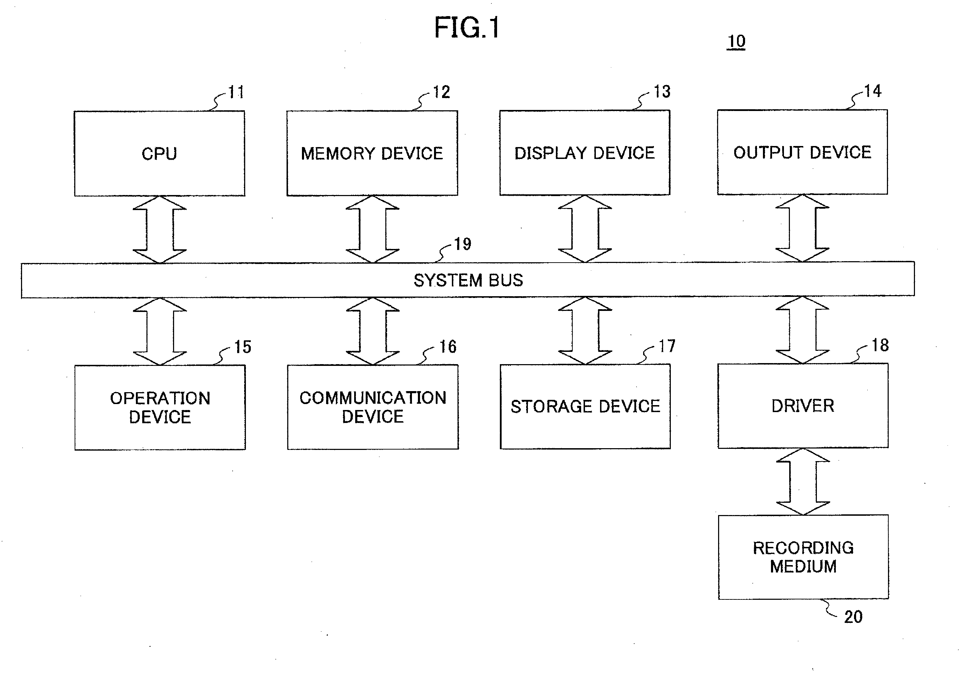

In the following, embodiments of the present invention are described with reference to the accompanying drawings. The processes for constructing a new molecular structure are performed with a computer 10 illustrated in Fig. 1.

Fig. 1 is a schematic diagram illustrating a hardware configuration of the computer 10 for constructing a molecular structure (molecular structure construction) according to an embodiment of the present invention. Various scientific calculation processes are performed by using the computer 10. For example, the computer 10 may be used to perform molecular structure construction in which molecular attributes of a molecule can be obtained by performing molecular orbital calculation on the molecule by using a dedicated program.

In Fig. 1, the computer 10 includes, for example, a CPU (Central Processing Unit) 11, a memory device 12, a display device 13, an output device 14, an operation device 15, a communication device 16, a storage device 17, and a driver 18 that are connected by a system bus 19.

The CPU 11 entirely controls the computer 10 in accordance with a program stored in the memory device 12. The memory device 12 stores, for example, a program(s) executed by the CPU 11, data required for processes performed by the CPU 11, and data obtained by the processes performed by the CPU 11. For example, a RAM (Random Access Memory), a ROM (Read Only Memory), or the like may be used as the memory device 12. Further, a portion of the memory device 12 is assigned to be used as a work area at which the processes of the CPU 11 are performed.

The display device 13 displays various data in accordance with the control of the CPU 11. The output device 14 outputs various data in accordance with instructions from the user. The output device 14 may be, for example, a printer. The operation device 15 is operated by the user for inputting data for causing the computer 10 to perform various processes. The operation device 15 may be, for example, a keyboard, a pointing device (e.g., mouse), or the like. The below-described GUI can be configured by interaction between the above-described units of the computer 10 including the display device 13, the operation device 14, and the CPU 11.

The communication part 16 controls communication with respect to external devices by connecting to a network such as the Internet, a LAN (Local Area Network), or the like. The storage device 17 stores, for example, programs for performing various processes and various data. For example, a hard disk unit may be used as the storage device 17.

The program used for performing a process by the computer 10 may be provided to the computer 10 from a recording medium 20 such as a CD-ROM (Compact Disc Read-Only Memory). When the recording medium 20 on which the program is recorded is set to the driver 18, the driver 18 reads out the program from the recording medium 20. Then, the program is installed in the storage device 17 via the system bus 19. When the program is activated, the CPU 11 initiates the process in accordance with the program stored in the storage device 17. It is to be noted that the recording medium 20 on which the program is recorded is not limited to a CD-ROM. Other media may be used as the recording medium 20 as long as the program can be read out by the computer 10. Alternatively, in a case where the computer 10 is compatible to USB (Universal Serial Bus), the program may be installed from an external storage device that is connectable to USB.

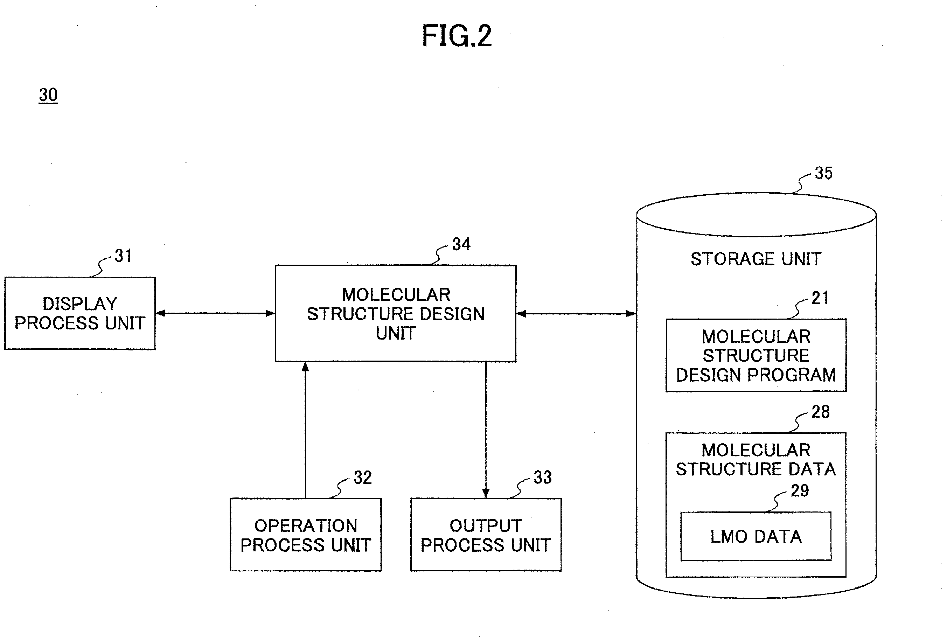

The below-described molecule structure construction assisting apparatus 30 according to an embodiment of the present invention includes an apparatus that can easily set an appropriate initial electron density matrix for enabling high speed molecular orbital calculation. More specifically, the molecule structure assisting apparatus 30 may include, for example, the above-described hardware configuration illustrated in Fig. 1 and the below-described functional configuration illustrated in Fig. 2.

(Configuration of molecule structure construction assisting apparatus)

In order to construct a LMO for only a changed part of a molecule by interactive GUI operation with data structures and configure an initial electron density matrix, the molecular structure construction assisting apparatus 30 according to an embodiment of the present invention has a functional configuration illustrated in Fig. 2.

According to the functional configuration of Fig. 2, the molecular structure construction assisting apparatus 30 includes, for example, a display process unit 31, an operation process unit 32, an output process unit 33, a molecular structure design unit 34, and a storage unit 35.

The functions (processes) of the display process unit 31, the operation process unit 32, the output process unit 33, and the molecular structure design unit 34 are performed by having the CPU 11 of the computer 10 execute programs corresponding to display process unit 31, the operation process unit 32, the output process unit 33, and the molecular structure design unit 34. Thus, the computer 10 functions as the molecular structure construction assisting apparatus 30 according to an embodiment of the present invention.

The storage unit 35 includes, for example, the storage device 17 and/or the memory device 12. The storage unit 35 stores various data according to an embodiment of the present invention. At least a molecular structure design program 21 and molecular structure data 28 including LMO data 29 are stored in the storage unit 35. The function of the molecular structure design unit 34 is performed by having the CPU 11 execute the molecular structure design program 21.

The display process unit 31 controls data display performed by the display device 13. The operation process unit 32 controls data input from a pointing device operated by the user. The output process unit 33 controls data output performed by the output device 14.

The molecular structure design unit 34 performs a process related to the below-described molecular structure design. The molecular structure design unit 34 stores the molecular structure data 28 including the LMO data 29 by using the below-described data structure 40. By using the molecular structure data 28 including the LMO data 29 for performing a molecular orbital calculation process, molecular orbital can be calculated efficiently and effectively.

The process related to the molecular structure design is performed by performing, for example, an operation (including GUI operation) using the molecular structure construction assisting apparatus 30. The user selects and allocates an atomic item of a three-dimensional molecular structure by operating (e.g., clicking, dragging & dropping) a pointing device or a keyboard on a main window displayed on the display device 13 of Fig. 1 by the display process unit 31.

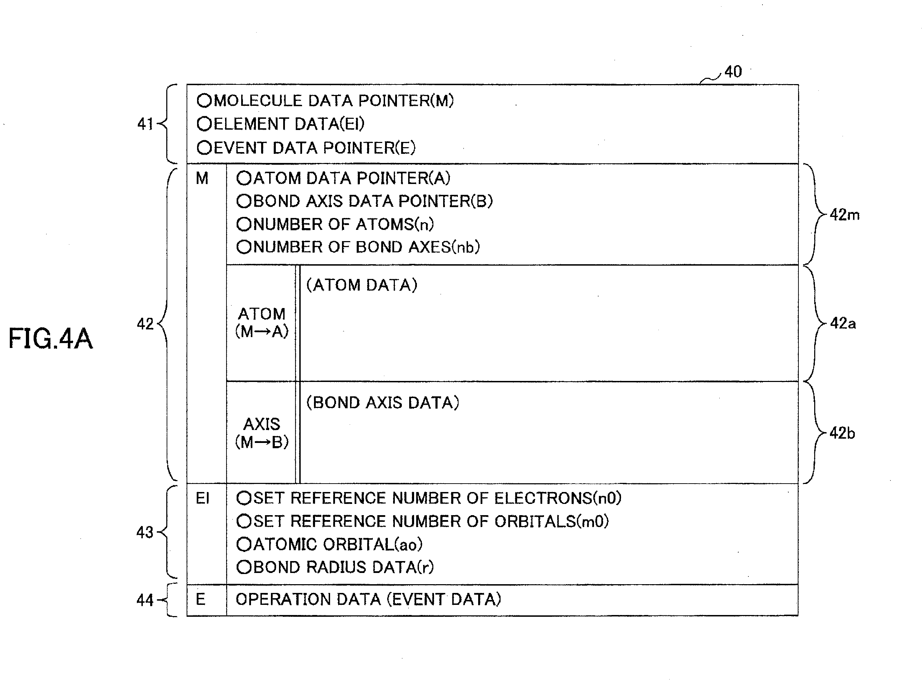

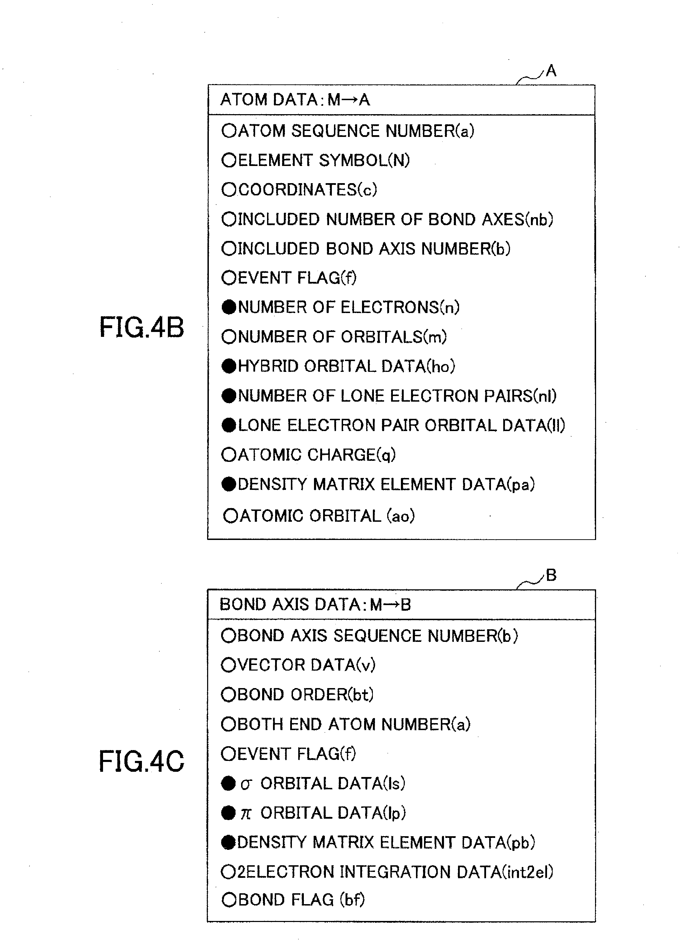

The displayed data such as the atomic item of the three-dimensional structure configured by the aforementioned operation is stored beforehand in an external storage device (e.g., HDD (Hard Disk Drive)) or the storage unit 35. Then, the operation process of the operation process unit 32 is processed by the molecular structure design unit 34. Then, the three-dimensional molecular structure is displayed by the display process unit 31 together with the course (process) of selecting and allocating the atomic item of the three-dimensional molecular structure. The three-dimensional molecular structure of the molecule displayed on the GUI includes the below-described data structure illustrated in Fig. 4. The operations controlled by the operation process unit 32 include a process of storing data pertaining to construction and change of the data structure into the storage unit 35.

Then, the atomic item selected from the screen of the display device 13 with the operation process unit 32 is changed or re-allocated by inserting (inputting) the atomic item to a desired location. By the GUI operation, a new molecular structure including the atomic item of the three-dimensional molecular structure is configured, and re-construction of various data structures (including the below-described data structure 40 according to an embodiment of the present invention) is performed inside the storage unit 34.

Then, the molecule structure design part 34 performs a process for constructing an LMO for only a changed part of a changed molecular structure by using a program according to an embodiment of the present invention. Thereby, density matrix element data of the below-described atom data structure A (i.e. electron density data of atom) and density matrix element data of the below-described bond axis data structure B (i.e. electron density data between atoms) are calculated. The molecule structure design part 34 performs a changing process only to a part of the initial electron density matrix required to be updated based on the calculated density matrix element data and determines the initial electron density matrix.

(Flow of LMO construction)

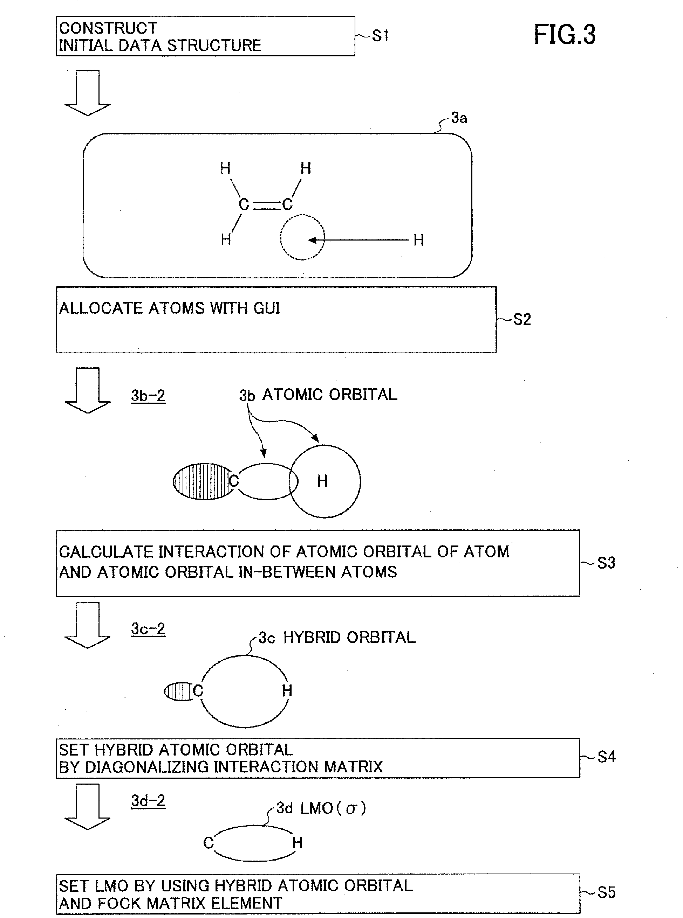

More specifically, in calculating the initial electron density according to an embodiment of the present invention, attention is drawn to a connecting part between newly set atoms. Based on the Fock element and the hybrid orbital regarding the connecting part, LMO is derived. Then, the initial electron density is calculated based on the derived LMO. Next, the flow for constructing the LMO with the electron structure design unit 34 is described with reference to Fig. 3.

Fig. 3 illustrates the flow of LMO construction where the molecular structure construction assisting apparatus 30 according to an embodiment of the present invention constructs an LMO only for a connecting part of a changed atom(s) of a new molecule (e.g., a molecule having an atom added to a portion thereof).

As illustrated in Fig. 3, first, the molecular structure construction assisting apparatus 30 according to an embodiment of the present invention constructs the below-described data structure 40 by storing initial values of the data structure 40 required for calculation into the storage unit 35 by using, for example, a recording medium having data recorded thereon (Step S1).

Then, an atom(s) is allocated with a GUI by performing interactive operation between the operation device 15 and the display device 13 (Step S2). For example, reference numeral 3a of Fig. 3 illustrates a screen in a case where a hydrogen (H) atom is allocated to a $C_2H_3$ molecule with the GUI. In Step S2, the distance between atoms is determined along with determining whether there is a formation of a bond (bond formation). In a case where the molecular structure assisting apparatus 30 determines that there is bond formation, the flow proceeds to Step S3.

Then, based on the connection of a carbon (C) atom with respect to the newly added H atom, the molecular structure design unit 34 calculates the interaction between the atomic orbital of the atoms constituting the bond and the atomic orbital in-between the atoms (see, for example, the state indicated with reference numeral 3b-2 in Fig. 3). The calculation of the interaction corresponds to the calculation of 2 election integration and Fock elements with respect to the part related to the bond (Step S3).

Then, as indicated with reference numeral 3c-2 in Fig. 3, the molecular structure design unit 34 performs calculation of the hybrid (atomic) orbital 3c by diagonalizing the interactive matrices (Step S4).

Then, as indicated with reference numeral 3d-2 in Fig. 3, the molecular structure design unit 34 derives LMO (σ) 3d by using the hybrid (atomic) orbital 3c and the Fock element (Step S5). In performing Step S5, σ LMO and π LMO are taken into consideration.

(Data structure)

Next, the data structure 40 of the molecular structure data 28 used in the processes performed by the molecular structure construction assisting apparatus 30 is described. Figs. 4A-4C are schematic diagrams for describing the data structure 40 according to an embodiment of the present invention. The data structure 40 illustrated in Fig. 4A may be set by being loaded in the storage unit 35 and storing data in accordance with a molecular structure construction assisting process. The data structure 40 may be used for referring to the molecular structure data 28 stored in the storage unit.

In Fig. 4A, the data structure 40 includes, for example, a header 41, molecule data 42, element data 43, and event data (operation data). The molecule data 42 includes, for example, molecule main data 42m, atom data 42a, and bond axis data 42b.

The header 41 includes, for example, a data structure having a molecule data pointer (M), an element data pointer (El), and an event data pointer (E). The molecule data pointer (M) is a pointer that indicates a top address at which the molecule data 42 is stored. The element data pointer (El) is a pointer that indicates a top address at which the element data 43 is stored. The event pointer (E) is a pointer that indicates a top address at which the event data 44 is stored.

The molecule main data 42m of the molecule data 42 has a data structure including, for example, an atom data pointer (A), a bond axis data pointer (B), data pertaining to the number of atoms (n), and data pertaining to the number of bond axes (nb). The atom data pointer (A) is a pointer that indicates a top address at which the atom data 42a is stored. The bond axis data pointer (B) is a pointer that indicates a top address at which the bond axis data 42b is stored.

The number of atoms (n) is the number of atoms managed in the molecule data 42. That is, the number of atoms (n) is the number of atoms allocated by user with the GUI and corresponds to the number of atom data 42a. The number of bond axes (nb) is the number of bond axes managed in the molecule data 42 and corresponds to the number of bond axis data 42b.

According to an embodiment of the present invention, the data structure of the molecule data 42 is assumed as "molecule data structure M", the data structure of the atom data 42a is assumed as "atom data structure A", and the data structure of the bond axis data 42b is assumed as "bond axis data structure B".

Further, the data structure of the element data 43 is assumed as "element data structure El". One of the characteristics of the data structure 40 according to an embodiment of the present invention is that each of the atom data structure A of the atom data 42a and the bond axis data structure B of the bond axis data 42b include data (data items) establish a one-on-one relationship with respect to all of the atoms and bond axes.

The atom data 42a also includes, for example, data pertaining to the number of orbitals and the number of electrons accompanying the atom, flag data required for calculation, hybrid orbital (HO) data, lone electron pair LMO data, data pertaining to the number of lone electron pairs, and atomic charge data.

First, the items (elements) of the atom data structure A of the atom data 42a included in the data structure 40 of Fig. 4A are described with reference to Fig. 4B. The atom data structure A illustrated in Fig. 4B includes items such as atom sequence number (a), element symbol data (N), coordinate data (c), included number of bond axes (nb), included bond axis number (b), event flag (f), number of electrons (n), number of orbitals (m), hybrid orbital data (ho), number of electron pairs (nl), lone electron pair orbital data (11), atomic charge (q), density matrix element data (pa), and atom orbital (a0).

Next, the items (elements) of the bond axis data structure B of the bond axis data 42b included in the data structure 40 of Fig. 4A are described with reference to Fig. 4C. The bond axis data structure B illustrated in Fig. 4C includes items such as bond axis sequence number (b), vector data (v), bond order (bt), both end atom number (a), event flag (f), σ orbital data (ls), σ orbital data (lp), density matrix element data (pb), 2 electron integration data (int2el), and bond flag (bf).

The element data structure El of the element data 43 included in the data structure 40 of Fig. 4A includes items such as bond radius (r), reference number of electrons (nO), and reference number of orbitals (m0).

For example, with the above-described data structure 40, the hybrid orbital data (ho) of the atom data structure A can be expressed with a pointer "A->ho". For example, the element data structure El of the element data 43 includes items of at least bond radius (r), reference number of electrons (nO), reference number of orbitals (m0), and atomic orbital (a0) that correspond to each element (indicated as "i"). The bond radius (r) can be expressed with a pointer "El [i] ->r", the reference number of electrons (nO) can be expressed with a pointer "El [i] ->n0", the reference number of orbitals (m0) can be expressed with a pointer "El [i] ->m0", and the atomic orbital (a0) can be expressed with a pointer "El [i] ->a0".

Further, in the atom data structure A illustrated in Fig. 4B, the atom sequence number "A->a" serves as an identification number in an internal coordinate system. The element symbol data "A->N" indicates an element symbol or an atom number of an element. The coordinate data "A->c[3]" indicates a position coordinate component in a three-dimensional coordinate system of each atom. The included bond axis number "A->nb" indicates the number of bond axes of an atom with respect to another atom(s). The value of the included bond axis number "A->nb" represents the number of bond axes estimated to constitute a pair of atoms according to the input molecular structure based on the atom radius "El->r". Further, the included bond axis number "A->b [nb]" indicates subset data of the identification number (included bond axis number) b of each bond axis in the included number of bond axes.

The event flag "A->f" is a flag representing various statuses (e.g., whether LMO is already set). For example, as explained in the process described with reference to Fig. 3, the number of electrons "A->n" may change from an initial value (value of set reference number of electrons) "El->n0" to "0". For example, as explained in the process described with reference to Fig. 3, the number of orbitals "A->m" may also change from an initial value (value of set reference number of electrons) "El->m0" to "0". The hybrid orbital data "A->ho" indicates data pertaining to hybrid orbital (HO), the lone electron pair "A->n1" indicates the number of lone electron pairs, and the lone electron pair orbital data "A->11" indicates data pertaining to lone electron pair orbital data (particularly, LMO data). The atomic charge "A->q" indicates the charge(s) on an atom.

The bond flag "A->bf" is for indicating whether a bond axis can be set. For example, the initial value of the bond flag (bf) is set to "F". In a case where the number of orbitals "A>m" and the number of electrons "n" are 0, the value of the bond flag is "F". In a case where either the number of orbitals "A>m" or the number of electrons "n" is not 0, and there is one bond in which the bond flag "A->bf" of another atom j related to the included bond axis number "A->b" is "F" while the other remaining bond flags are "T", the value of the bond flag is "C". During operation, the value of the bond flag is also "C" in a case where there is one bond in which the bond flag "A->bf" of the other atom j is "C" while the other remaining bond flags are "T".

In the bond axis data structure B illustrated in Fig. 4C, the bond axis sequence number "B->b" serves as an identification number corresponding to each bond axis for establishing a one-on-one relationship with respect to each bond axis. The vector data "B->v" indicates a three-dimensional vector corresponding to each bond axis for establishing a one-on-one relationship with respect to each bond axis. The initial value of the bond index "B->bt" is 0. For example, the bond index "B->bt" may change to an appropriate bond order according to the above-explained process described with reference to Fig. 3.

The both end atom number "B->a [2]" indicates subset data of the atom sequence number (=atom identification number) existing on both ends of a bond axis. The event flag "B->f" is a flag representing various statuses (e.g., progress of the procedures in the process described with reference to Fig. 3). The σ orbital data "B->1s" indicates LMO data of the σ bond, and the π orbital data "B->pb" indicates LMO data of the π bond. Further, the 2 electron integration data "B->int2el" indicates data for storing 2 electron integration between 2 atoms.

The data structure M of the molecule data 42 (see, for example, Fig. 4A) includes the atom data (structure) pointer "M->A" and the bond axis data (structure) pointer "M->nb" that have data related to each other. In order to perform the process illustrated in Fig. 3, the data structure M of the molecule data 42 also includes the number of atoms "M->n" and the number of bond axes "M->nb".

The element data structure EI of the element data 43 (see, for example, Fig. 4A) includes the reference number of electrons "EI->n0", the reference number of orbitals "EI->m0", the atomic orbital data "EI->a0", and the bond radius data "E1->r".

As illustrated in Fig. 4B, the atom data structure A of the data structure 40 includes the density matrix element data "A-pa" (electron density data on an atom). As illustrated in Fig. 4C, the bond axis data structure B of the data structure 40 includes density matrix element data "B-pb" (electron density data in-between atoms). After constructing LMO data with the atom data structure A and the bond axis data structure B, only necessary electron density data can be updated by using the changed LMO data, and electron density data can also be established.

In a case of performing molecular structure design on a molecule having an odd number of electrons, the items indicated with a ● (black circle) in Fig. 4B (e.g., number of electrons, (n), hybrid orbital data (ho), number of lone electron pairs (nl), lone electron pair open data (11), density matrix element data (pa)) may be arranged and doubled (duplex) with respect to up-spin electrons and down-spin electrons.

In this case, the items of the bond axis data structure B indicated with a ● (black circle) in Fig. 4C (e.g., σ orbital data (ls), π orbital data (lp), density matrix element data (pb)) may be arranged and doubled.

The lone electron pair orbital data (11) of the atom data structure A (Fig. 4B) and the σ orbital data (ls) and π orbital data (lp) of the bond axis data structure B (Fig. 4C) correspond to the LMO data 29 illustrated in Fig. 2.

(Initialization of data structure)

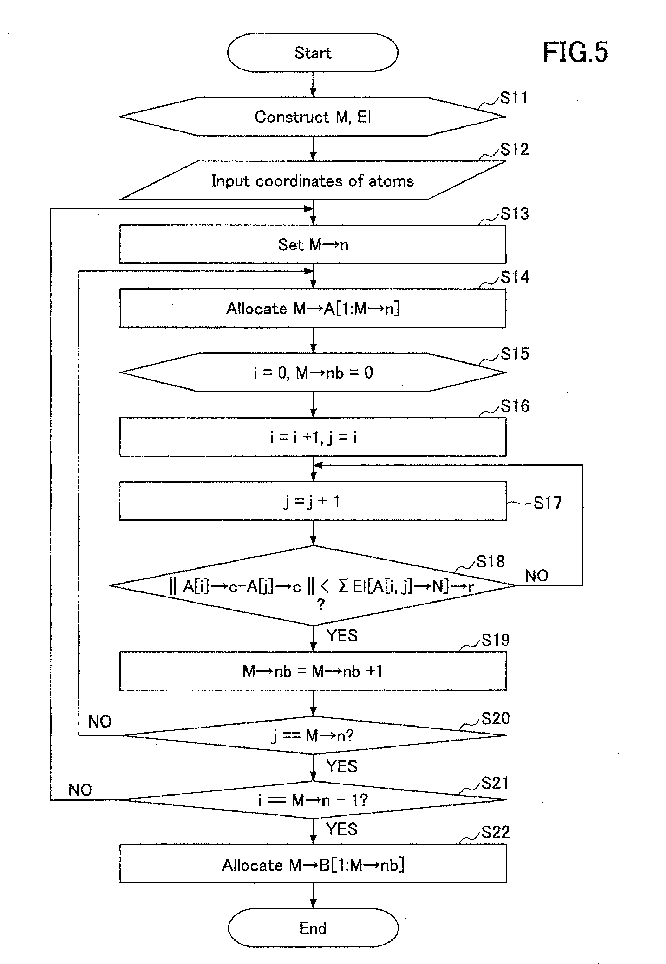

First, in constructing the above-described data structure 40, initialization of data structure is performed. According to an embodiment of the present invention, in a case where the above-described GUI is operated to form a new bond by adding, removing, or changing an atom with respect to an existing molecular structure, the interaction between the atomic orbital of the atom constituting the new bond and the atomic orbital in-between the atoms is calculated. Fig. 5 illustrates the flow of an initialization process for initializing a data structure required for the calculation.

In Fig. 5, the molecular structure design unit 34 of the molecular structure construction assisting apparatus 30 constructs the molecule data structure M of the molecule data 42 and the element data structure EI of the element data 43 (Step S11).

In a case of setting the element data structure EI (e.g., setting the element data structure from atom number 1 of hydrogen to atom number 88 of radium), a sufficient memory space is acquired for EI [1:88]. A value corresponding to each element is set to data items including, for example, the atom radius, the reference number of electrons, the reference number of orbitals, and the atomic orbital. For example, van der waals radius of each element is set to the atom radius "EI->r". Further, the reference number of electrons "EI->n0" and the reference number of orbitals "EI->m0" are set with a value taking a basis function used in molecular orbital calculation into consideration. Further, the atom orbital "EI->a0" is also set with a value taking the basis function into consideration. It is to be noted that the data of the basic function and the basic function to be used are loaded from corresponding files of the basic function by operating the GUI of the molecular structure construction assisting apparatus 30.

The molecular structure design unit 34 acquires (allocates) a sufficient memory space for the pointer "M->A" to the atom data structure A (Fig. 4B) and for the pointer "M->B" to the bond axis data structure B (Fig. 4C). Further, the molecular structure design unit 34 also acquires (allocates) a memory space for all of the number of atoms "M->n" and all of the number of bond axes "M->nb". The pointers "M->A" and "M->nb" may be set according to circumstance.

Then, the molecular structure design unit 34 inputs (three-dimensional) position coordinates of an atom (Step S12). Then, the molecular structure design unit 34 sets the number of atoms "n" to the item "M->n" based on the number of input position coordinates of the atom (Step S13). Then, the molecular structure design unit 34 acquires (allocates) a memory space for the molecule data structure "M->A [1:M->n]" (Step S14) in a number equivalent to the number of atoms n (Step S14).

Then, the molecular structure design unit 34 sets 0 to the variable designating the sequence of an atom (atom sequence variable i, hereinafter also referred to as atom i) and sets 0 to the bond axis number "M->nb" (Step S15). In Step S16, the molecular structure design unit 34 increments the atom i by adding 1 to the atom i and designates the next atom. Further, in Step S16, the molecular structure design unit 34 sets "i" to an atom sequence variable j (a variable to be bonded to the atom i, hereinafter also referred to as atom j). Further, the molecular structure design unit 34 increments the atom j by adding 1 to the atom j (target bond atom) (Step S17).

Then, the molecular structure design unit 34 determines whether the distance between the atom i and the atom j is less than a threshold that is determined in accordance with each bond radius r (Step S18). For example, the molecular structure design unit 34 determines whether the difference (absolute value) between the coordinates of the atom i and the coordinates of the atom j is less than the bond radius r of each element. In a case where the distance between the atom i and the atom j is equal to or greater than the threshold (No in Step S18), the molecular structure design unit 34 determines that the atom i is not to be bonded to the atom j and returns to Step S17 for repeating the processes described above. On the other hand, in a case where the distance between the atom i and the atom j is less than the threshold (Yes in Step S18), the molecular structure design unit 34 increments the bond axis number "M->nb" by adding 1 to the bond axis number (Step S19).

Then, the molecular structure design unit 34 determines the completion of processing the atom i with respect to the atom j (target bond atom) equivalent to the number of atoms "M->n". That is, the molecular structure design unit 34 determines whether the atom j matches the number of atoms n (Step S20).

In a case where the processing of the atom i is not completed (No in Step S20), the molecular structure design part 34 returns to Step S13 and repeats the processes described above. In a case where the processing of all of the atoms i is completed (Yes in Step S20), the molecular structure design unit 34 determines the completion of processing all of the atoms i equivalent to a value obtained by subtracting 1 from the number of atoms "M->n". That is, the molecular structure design unit 34 determines whether the atom i matches a value obtained by subtracting 1 from the number of atoms n (Step S21).

In a case where the processing is not completed (No in Step S21), the molecular structure design unit 34 returns to Step S13 and continues the processes described above. Because the number of all of the bond axes are determined when the processing of all of the atoms i is completed (Yes in Step S21), the molecular structure design unit 34 allocates a memory space for the bond axis data structure "M->B[1:M->nb]" in a number equivalent to the number of bond axes nb (Step S22) and terminates the operation (flow) of initializing the data structure. The identification number for identifying the atoms and the bond axes may be set in accordance with the sequence (order) in which the atom or the bond axis is determined. That is, the identification number for identifying the atoms and the bond axes may be set with the atom sequence number "A->a" and the bond axis sequence number "B->b", respectively.

(Setting of localized molecular orbital (LMO))

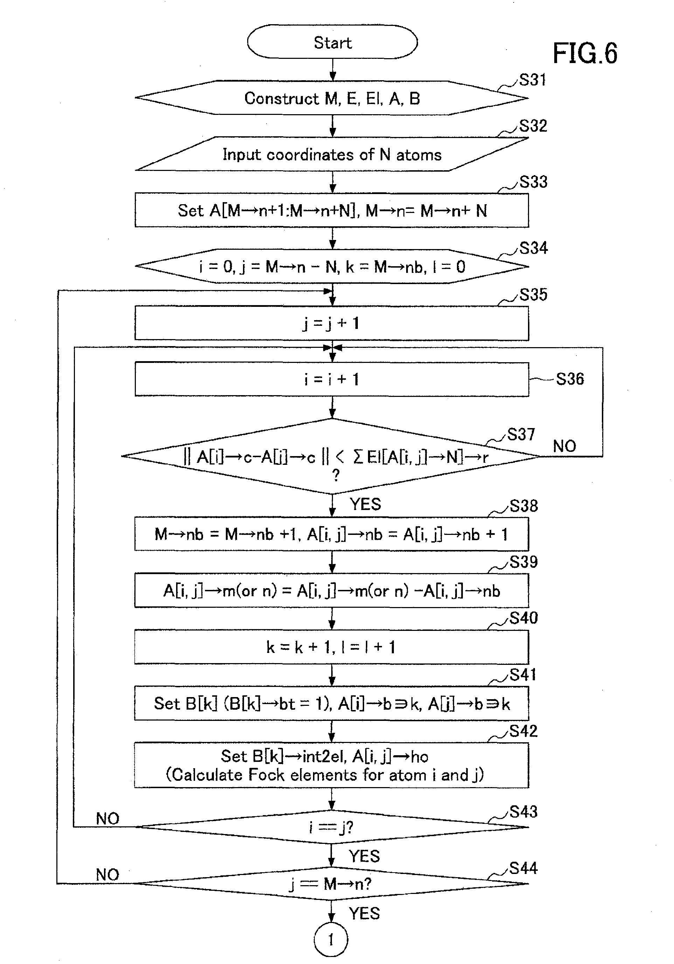

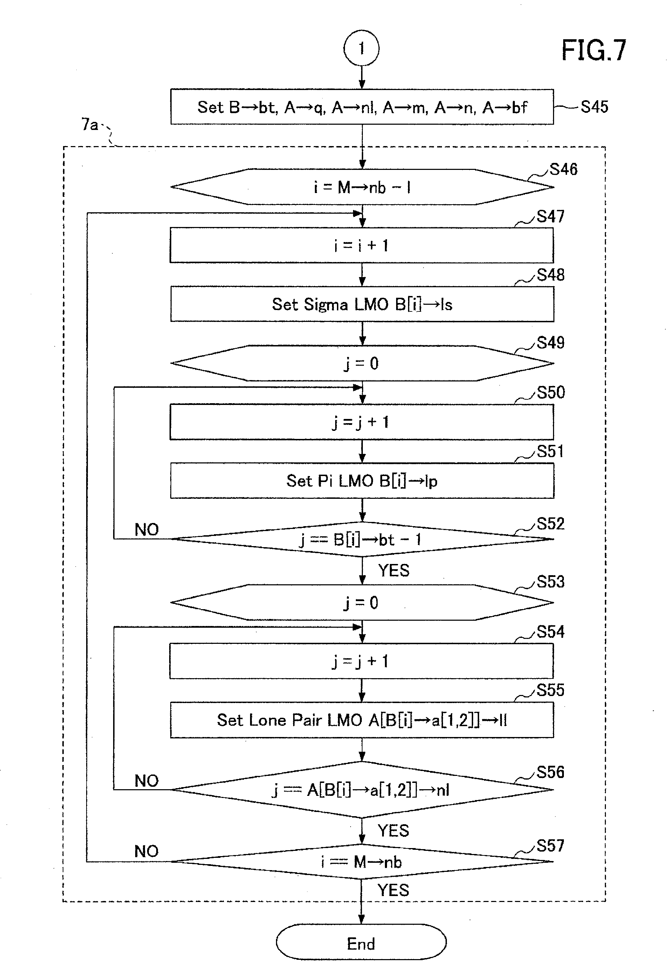

After the initialization of data structure, the setting (deriving) of LMO is performed. The process of setting LMO is described with Figs. 6 and 7. The LMO setting process according to an embodiment of the present invention includes steps S32-S57 illustrated in Figs. 6 and 7. With the LMO setting process illustrated in Figs. 6 and 7, the LMO is set by calculating the interaction between the atomic orbital of the atom constituting the bond and the atomic orbital in-between the atoms. The following LMO setting process is described with an example where N atoms are added to a molecular structure.

In Fig. 6, the molecular structure design unit 34 of the molecular structure construction assisting apparatus 30 constructs the molecule data structure M, the event data structure (operation data structure) E, the element data structure El, the atom data structure A, and the bond axis data structure B (Step S31). First, except for the element data structure and the molecular data structure initialized beforehand, the molecular structure design unit 34 initializes the atom data structure A and the bond axis data structure B.

An input sequence number is set to the atom sequence number "A[i]->a" of the atom sequence variable i (hereinafter also referred to as "i").

An atom number of an element is set to the element symbol "A[i]->N" of the atom i. Cartesian coordinates of an input atom is set to the coordinates "A[i]->c" of the atom i. The reference number of electrons of an element symbol N is set to the initial number of electrons of the atom i (that is, "A[i]->n = El [A[i]->n0"]. The reference number of orbitals of the element symbol N is set to the initial number of orbitals of the atom i (that is, "A[i]->n = El [A[i]->N] ->m0"). Further, 0 is set to the initial atomic charge of the atom i (that is, "A[i]-q = 0"). The included number of bond axes of the atom i (A[i]->nb) and the included bond axis number of the atom i (A[i]->b) are set at the same time of initializing the bond axis data structure B.

Then, the molecular structure design unit 34 inputs the (three-dimensional) position coordinates of N atoms (Step S32). Then, in Step S33, the molecular structure design unit 34 allocates a memory space for the atom data structure A in a number equivalent to the number of atoms ranging from "M->n+1" to "M->n+N" based on the number of position coordinates of the input atoms. Further, in Step S33, the molecular structure design unit 34 sets the number of atoms "M->n" to "M->n + N".

Then, the molecular structure design unit 34 sets 0 to the atom i, sets the number of atoms "M->n-N" to the atom j, sets the bond axis sequence variable k (hereinafter also referred to as "bond axis k") to the included number of bond axes "M->nb", and sets 0 to the number of bond axis to be added "1" (Step S34).

Then, the molecular structure design unit 34 increments the atom j to j + 1 by adding 1 to the atom j (Step S35). Then, the molecular structure design unit 34 increments the atom i to i + 1 by adding 1 to the atom i (Step S36).

Then, as described in detail below, the molecular structure design unit 34 determines whether the distance between the atom i and the atom j (target bond atom) is equal to or less than a threshold and sets the bond axis data structure B to the bond between the atoms i and j if the distance is equal to or less than the threshold.

In Step S37, the molecular structure design unit 34 determines whether the distance between the atom i and the atom j is less than the threshold determined according to each bond radius r. More specifically, the molecular structure design unit 34 determines whether the difference between the coordinates c of the atom i and the coordinates c of the atom j (absolute value of the difference) is less than each of the bond radiuses of the elements that include the atoms i and j. In a case where the difference is equal to or greater than the threshold (No in Step S37), the molecular structure design unit 34 does not bond the atom i to the atom j and returns to Step S36 for repeating the processes described above. On the other hand, in a case where the difference is less than the threshold (Yes in Step S37), the molecular structure design unit 34 increments the number of bond axes "M->nb" by adding 1 to the number of bond axes and increments the included number of bond axes "A[i, j] - >nb" including the atoms i and j by adding 1 to the included number of bond axes (Step S38).

Then, the molecular structure design unit 34 sets the number of bond axes including the atoms i and j or the number of electrons "A [i, j] ->m (or n) to a value obtained by subtracting the included number of bond axes "A [i, j] ->nb" including the atoms i and j from the number of bond axes including the atoms i and j or the number of electrons "A [i, j] ->m (or n) (Step S39).

Then, the molecular structure design unit 34 increments the bond axis k and the number of bond axes to be added 1 by adding 1 to the bond axis k and adding 1 to the number of bond axes to be added 1 (Step S40). Then, the molecular structure design unit 34 sets 1 to the bond order "B [k] - > bt", adds the included bond axis number b of the atom i to the bond axis k, and adds the included bond axis number b of the atom j to the bond axis k (Step S41).

For example, when the distance between the atom i and the atom j is determined to be less than the threshold in a state where the k-1 bond axes are already set, the bond axis between the atom i and the atom j is set with the bond axis data structure B[k] of the bond axis k. In this example, k is set to the included number of bond axes nb (i.e. B[k]->nb = k); i is set to one atom sequence number (i.e. B[k]->a[1] = i); j is set to another atom sequence number (i.e. B[k]->a[2] = j); the included number of bond axes "A[i]->nb" of the atom i is incremented by adding 1 to the included number of bond axes of the atom i; the included number of bond axes "A[i]->b" of the atom i is set to "k"; and the included number of bond axes "A[j]->b" of the atom j is set to k.

The initialization of the hybrid orbital data (HO data) including the atoms i and j "A[i, j]->N]" is performed with respect to the atomic orbital EI of element symbol N "[A[i,j]->N]->a0". The bond order "B[i,j]->bt" may be designated directly by operating the GUI.

Then, as described in detail below, calculation of the interaction between the atomic orbital of the atoms constituting the bond and the atomic orbital in-between the atoms is performed. In Step S42, the molecular structure design unit 34 sets 2 electron integration data between one both end electron number "B[k]->a[1]" and another both end electron number "B[k]->a[2]" to the 2 electron integration data of the bond axis data structure B of the bond axis k "B[k]->int2el". Further, in Step S42, the molecular structure design unit 34 calculates the Fock elements with respect to the atoms i and j. In calculating the interaction matrix, the 2 electron integration data is set to the Hamiltonian element between the atoms. The Hamiltonian element may be read by using the same method used for regular molecular orbital calculation. When the interaction matrix is obtained, a unique vector can be obtained by diagonalization. Then, by performing orthogonal transformation on the unique vectors, hybrid orbital data (HO data) can be obtained.

Then, the molecular structure design unit 34 determines whether the atom i matches the atom j (Step S43). In a case where the atom i does not match the atom j (No in Step S43), the molecular structure design unit 34 returns to Step S36 and repeats the above-described processes. On the other hand, in a case where the atom i matches the atom j (Yes in Step S43), the molecular structure design unit 34 determines whether the atom j matches the number of atoms "M->n" (Step S44).

In a case where the molecular structure design unit 34 determines that the atom j does not match the number of atoms "M->n" (No in Step S44), the molecular structure design unit 34 returns to Step S35 and repeats the above-described processes. In a case where the molecular structure design unit 34 determines that the atom j matches the number of atoms "M->n" (Yes in Step S44), the molecular structure design unit 34 sets the bond order of the bond axis data structure B "B->bt", the atomic charge of the atom data structure A "A->q", the number of lone electron pairs of the atom data structure A "A->nl", the number of orbitals of the atom data structure A "A->m", the number of electrons of the atom data structure A "A->n", and the bond flag of the atom data structure A "A->bf" in Step S45 of Fig. 7.

In a case of designing a molecular structure of a molecule in which the number of electrons is an odd number, a process 7a including Steps S46-S57 is repeated with respect to up-spin electrons and down-spin electrons.

After Step S45, the molecular structure design unit 34 initializes the bond axis i with a value obtained by subtracting the number of added bond axes (in this example, 1) from the included number of bond axes "M->nb-1" (Step S46). Then, the molecular structure design unit 34 increments the bond axis i by adding 1 to the bond axis i (Step S47).

Then, the molecular structure design unit 34 sets the σ orbital data of the bond axis data structure B of the bond axis 1 "B[i]->ls" (Step S48). Then, the molecular structure design unit 34 initializes the counter of the bond order by setting 0 to the counter of the bond order (Step S49). It is to be noted that the σ orbital data may be indicated as σ LMO.

Then, the molecular structure design unit 34 increments the counter j by adding 1 to the counter j (Step S50). Then, the molecular structure design unit 34 sets the π orbital data of the bond axis data structure B of the bond axis i "B[i]->lp" (Step S51). It is to be noted that the π orbital data may be indicated as π LMO.

Then, in Step S52, the molecular structure design unit 34 determines whether the counter j matches a value obtained by subtracting 1 from the bond order of the bond axis i "B[i]->bt-1". That is, in Step S52, the molecular structure design unit 34 detrmines whether the counter j has reached the number of π bonds "bt-1". In a case where the counter j does not match (No in Step S52), the molecular structure design unit 34 returns to Step S50 and repeats the above-described processes. In a case where the counter j matches (Yes in Step S52), the molecular structure design unit 34 initializes the counter j (related to the number of lone electron pairs) by setting 0 to the counter j (Step S53) .

As described above, the σ orbital data B of the bond axis data structure B "B[i]->ls" is set to the LMO of the σ bond. More specifically, a single orbital is obtained by applying a coefficient obtained from a solution of a two-dimensional array formed of an integrated value of the Fock element and the hybrid orbital data to the hybrid orbital of the atoms on both ends of the bond axis. Thereby, the σ LMO can be obtained. The hybrid orbital data used in this example is a hybrid orbital data that maximizes the interaction. The LMO of the π bond is obtained substantially in a similar manner as the σ LMO in which a remaining orbital data is used with respect to a value obtained by subtracting 1 from the bond order (equivalent to σ bond) "B[i]->bt-1".

Then, the molecular structure design unit 34 increments the counter j by adding 1 to the counter j (Step S54). Then, the molecular structure design unit 34 sets the atom numbers 1, 2 of both ends of the bond axis i to the lone electron pair orbital data "A [B [i]->a [1,2]]->11" (Step S55).

Then, in Step S56, the molecular structure design unit 34 determines whether the counter j matches the number of lone electron pair orbitals of the atom numbers 1, 2 of both ends of the bond axis i "A [B [i]->a [1,2]]->nl". That is, in Step S56, the molecular structure design unit 34 determines whether the counter j has reached the number of lone electron pairs nl. Then, in a case where the counter j does not match (No in Step S56), the molecular structure design unit 34 returns to Step S54 and repeats the above-described processes. In a case where the counter J matches (Yes in Step S56), the molecular structure design unit 34 determines whether the bond axis i matches the number of bond axes "M->nb" (Step S57). That is, in Step S57, the molecular structure design unit 34 determines whether the bond axis i has reached the number of bond axes nb. Then, in a case where the bond axis i does not match (No in Step S57), the molecular structure design unit 34 returns to Step S47 and repeats the above-described processes. In a case where the bond axis i matches (Yes in Step S57), the molecular structure design unit 34 terminates the operation (flow) of setting the LMO.

Accordingly, after setting the σ LMO and the π LMO, the molecular structure design unit 34 sets the remaining hybrid orbital (HO) and the number of the hybrid orbital to the lone electron pair orbital data "A [i, j]->11" and the number of lone electron pairs "A [i, j]->nl, respectively.

Accordingly, in a case where the density matrix element data of the atom i "A[i]->pa" and the density matrix element data of the bond axis k "B[k]->pb" are to be included in a data structure, not only LMO data but also initial electron density matrix data corresponding to each atom and each bond axis can be used on the GUI. The electron density matrix can be obtained by adding the products of the LMOs of occupied orbitals by using the σLMO, the n LMO, and the lone electron pair LMO.

(First example)

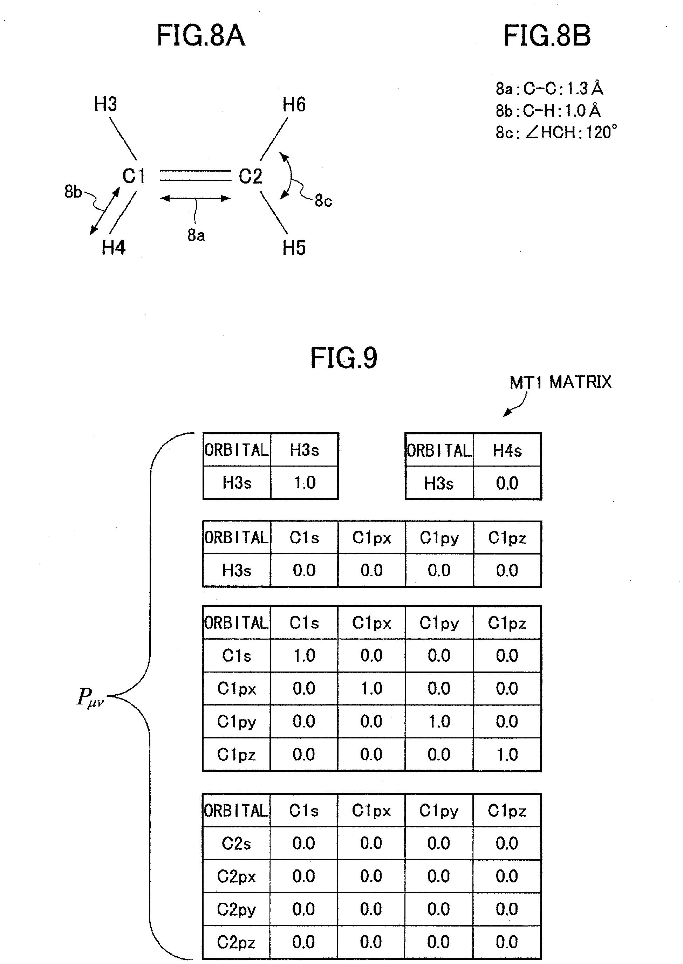

In the first example, the setting of LMO and initial electron density is performed on $C_2H_4$ (ethylene) by using the above-described processes according to an embodiment of the present invention. Figs. 8A and 8B are schematic diagrams for describing an example of a structure of $C_2H_4$ (ethylene). Fig. 8A illustrates a structure of $C_2H_4$ (ethylene). In Fig. 8A, numbers are assigned to atomic symbols (e.g., C1, C2, H3-H6) for identifying each atom.

A distance 8a between atoms C and C (C-C), a distance 8b between atoms C and H (C-H), and an angle 8c of H-C-H are illustrated in Fig. 8B. In this embodiment, the distance 8a between the atoms C and C is 1.3 angstroms, the distance 8b between the atom C and the atom H is 1.0 angstroms. Further, the angle 8c formed by the atoms H, C, and H is 120 degrees. In this example, a MNDO (Modified Neglect of Differential Overlap) method, which is a type of semiempirical molecular orbital method, is used as the method for performing molecular orbital calculation. In this example, the basic function is assumed as a slater-type orbital of the MNDO method.

The following results are obtained by having the CPU 11 execute the molecular structure design program 21 complying with the operation (flow) illustrated in Figs. 5-7. After the molecular structure and the initial data are set, the interaction between all atomic orbitals of C1 and the s orbital of other atoms are calculated as described below.

Although interaction is typically expressed with Fock elements, interaction is expressed with the following Formulas (1)-(3). The Fock elements pertaining to the orbitals $\mu$ of the atom A and the orbital $\mu$ of the atom A are expressed with Formula (1).

[Formula (1)]

$$F_{\mu\mu}^{AA} = H_{\mu\mu}^{core} + \sum_{\lambda \in A} P_{\lambda\lambda} \left\{ (\mu\mu|\lambda\lambda) - \frac{1}{2}(\mu\lambda|\mu\lambda) \right\} + \sum_{B \neq A} \sum_{\lambda,\sigma \in B} P_{\lambda\sigma} (\mu\mu|\lambda\sigma)$$

$$= H_{\mu\mu}^{core} + \sum_{\lambda \in A} P_{\lambda\lambda} \left\{ (\mu\mu|\lambda\lambda) - \frac{1}{2}(\mu\lambda|\mu\lambda) \right\} + \sum_{B \neq A} \sum_{\sigma \in B} P_{\sigma\sigma} (\mu\mu|\sigma\sigma)$$

The Fock elements pertaining to the orbitals $\mu$ of the atom A and the orbital $\nu$ of the atom A are expressed with Formula (2).

[Formula (2)]

$$F_{\mu\nu}^{AA} = H_{\mu\nu}^{core} + P_{\mu\nu} \left[ \frac{3}{2}(\mu\nu|\mu\nu) - \frac{1}{2}(\mu\mu|\nu\nu) \right] + \sum_{B \neq A} \sum_{\lambda,\sigma \in B} P_{\lambda\sigma} (\mu\nu|\lambda\sigma)$$

$$= H_{\mu\nu}^{core} + P_{\mu\mu}(\mu\mu|\mu\mu) + P_{\nu\nu}(\nu\nu|\nu\nu) + \sum_{B \neq A} \sum_{\sigma \in B} P_{\sigma\sigma} (\mu\nu|\sigma\sigma)$$

The Fock elements pertaining to the orbitals $\mu$ of the atom A and the orbital $\nu$ of the atom B are expressed with Formula (3).

[Formula (3)]

$$F_{\mu\nu}^{AB} = H_{\mu\nu}^{core} - \frac{1}{2} \sum_{\sigma \in A} \sum_{\lambda \in B} P_{\lambda\sigma} (\mu\sigma|\lambda\nu)$$

$$= H_{\mu\nu}^{core}$$

As illustrated in Formulas (1)-(3), matrices of core Hamiltonian H, 2 electron integration ($\mu\nu \mid \lambda\sigma$), and electron density P are used in order to calculate Fock elements.

In this example, definitions of each of the symbols in the Formulas (1)-(3) are depicted as follows.

$F_{\mu\nu}^{AB}$ $\Rightarrow$ A,B : ATOM, $\mu$, $\nu$ : ORBITAL, F: FOCK MATRIX (ELEMENT OF FOCK MATRIX PERTAINING TO ORBITAL $\mu$ OF ATOM A AND ORBITAL $\nu$ OF ATOM B)

$H_{\mu\nu}^{core}$ $\Rightarrow$ $\mu$, $\nu$ : ORBITAL , H$^{CORE}$: CORE HAMILTONIAN MATRIX (ELEMENT OF CORE HAMILTONIAN MATRIX PERTAINING TO ORBITAL $\mu$ AND ORBITAL $\nu$)

$P_{\mu\nu}$ $\Rightarrow$ $\mu$,$\nu$ : ORBITAL , P: ELECTRON DENSITY MATRIX (ELEMENT OF ELECTRON DENSITY MATRIX PERTAINING TO ORBITAL $\mu$ AND ORBITAL $\nu$)

$(\mu\nu \mid \lambda\sigma)$ $\Rightarrow$ $\mu$, $\nu$, $\lambda$, $\sigma$ : ORBITAL (2ELECTRON INTEGRATION BY CHEMIST'S NOTATION PERTAINING TO ORBITAL $\mu$, $\nu$, $\lambda$, $\sigma$)

Because the electron density matrix P is not yet set according to LMO in the calculations of Formulas (1)-(3), a diagonal matrix complying with the number of orbitals and electrons of ethylene is used as illustrated in the matrix MT1 of Fig. 9. The details of the abbreviations indicating the orbitals in the matrix MT1 of Fig. 9 are depicted as follows.

(Definitions I of abbreviations of orbitals of electron density matrix)

H3s: s orbital of third atom H

H4s: s orbital of fourth atom H

C1s: s orbital of first atom C

C1px: px orbital of first atom C

C1py: py orbital of first atom C

C1pz: pz orbital of first atom C

C2s: s orbital of second atom C

C2px: px orbital of second atom C

C2py: py orbital of second atom C

C2pz: pz orbital of second atom C

In describing the values of the matrix elements more specifically, the values of the elements between different atoms are all 0, and the values of the elements of the same orbital between the same atoms are equivalent to a value obtained by dividing the number of electrons included in the atom with the number of orbitals.

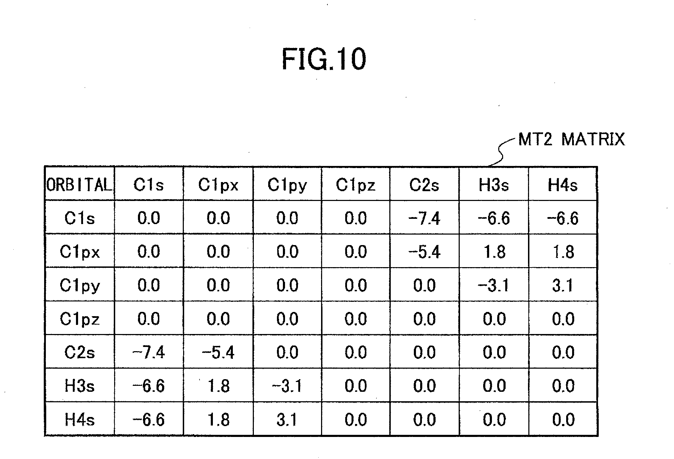

In calculating the Fock elements pertaining to interaction (Formula (3)), only the core Hamiltonian H of the elements between different atoms of the Fock elements need to be taken into consideration. An example of the Fock elements of the calculated interaction is illustrated in Fig. 10. The details of the abbreviations indicated in the orbitals of a matrix MT2 of Fig. 10 are the same as those of the above-described Definitions I.

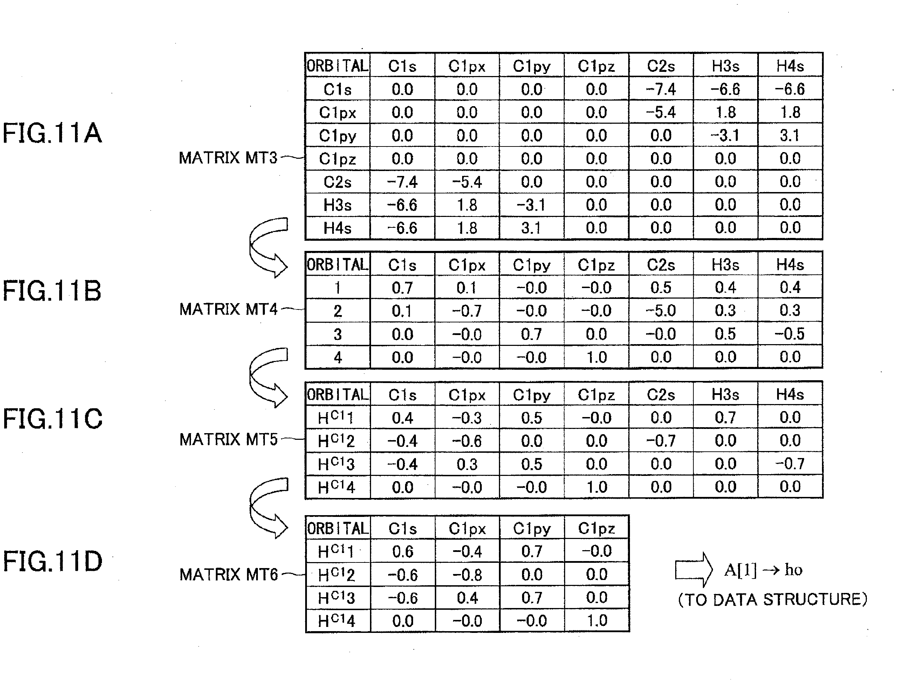

Figs. 11A-11D illustrate the flow for calculating the hybrid orbital (HO) used for configuring the localized molecular orbitals (LMO) where the hybrid orbital (HO) of atom C1 is calculated by using the interaction between the atomic orbital of C1 and the atomic orbital between other atoms. As illustrated in Fig. 11A, a matrix MT3 representing an interaction matrix is the same as the matrix MT2 illustrated in Fig. 10. Diagonalization process is performed on the matrix MT3 representing the interaction matrix. Thereby, as illustrated in Fig. 11B, data of a matrix MT4 representing unique vectors is obtained. Then, orthogonal transformation is performed on the matrix MT4 representing the unique vectors so that each of the elements of the other atoms corresponds to a single type of atom. As illustrated in Fig. 11C, as a result of performing the orthogonal transformation, a matrix MT5 representing hybrid orbitals between the atom C1 and another single type of atom is obtained. In reality, as illustrated in Fig. 11D, a matrix MT6 representing normalized hybrid orbitals are obtained by performing normalization only on the element of the atom C1. The normalized hybrid orbitals are stored in the hybrid orbital data of the atom data structure A "A[1]->ho" (see, for example, data structures of Figs. 4A and 4B).

In addition to the above-described Definition I indicating the details of the orbitals in each column of the matrices, details of the abbreviations indicating the configuration of the hybrid orbitals are depicted as follows.

(Definitions I of abbreviations of orbitals of electron density matrix)

HC11: first hybrid orbital of atom C1
$H^{C1}2$: second hybrid orbital of atom C1
$H^{C1}3$: third hybrid orbital of atom C1
$H^{C1}4$: fourth hybrid orbital of atom C1

Next, the flow of configuring the localized molecular orbitals (LMO) is depicted with Formulas (4)-(6) where the Fock elements calculated with Formulas (1)-(3) and the hybrid orbital data of the matrix MT5 of Fig. 11C are used.

[Formula (4)]

$$\begin{pmatrix} \left\langle H_\mu^A \left| F_{\mu\nu}^{AA} \right| H_\nu^A \right\rangle & \left\langle H_\mu^A \left| F_{\mu\nu}^{AB} \right| H_\nu^B \right\rangle \\ \left\langle H_\mu^B \left| F_{\mu\nu}^{BA} \right| H_\nu^A \right\rangle & \left\langle H_\mu^B \left| F_{\mu\nu}^{BB} \right| H_\nu^B \right\rangle \end{pmatrix} = \begin{pmatrix} E_{11} & E_{12} \\ E_{12} & E_{22} \end{pmatrix}$$

[Formula (5)]

$$\begin{vmatrix} E_{11} - \lambda & E_{12} \\ E_{12} & E_{22} - \lambda \end{vmatrix} = 0$$

[Formula (6)]

$$\begin{cases} \lambda_\pm = \dfrac{\left( E_{11} + E_{22} \right) \pm \sqrt{4E_{12}^2 + \left( E_{11} - E_{22} \right)^2}}{2} \\ \lambda' = \dfrac{E_{11} - E_{22}}{\left| E_{11} - E_{22} \right|} \sqrt{\left| E_{11} - \lambda_- \right|} \\ \lambda'' = \dfrac{-E_{12}}{\left| E_{12} \right|} \sqrt{\left| E_{22} - \lambda_+ \right|} \end{cases}$$

The LMOs of the bond between the atoms, which are derived by the Formulas (4)-(6), are illustrated in the following Formula (7).

[Formula (7)]

$$\begin{cases} LMO_{AB}^{Occupied} \equiv \left(\lambda' H^A\right) \cup \left(\lambda'' H^B\right) \\ LMO_{AB}^{Virtual} \equiv \left(\lambda'' H^A\right) \cup \left(-\lambda' H^B\right) \\ LMO_{A}^{LonePair} \equiv H^A \end{cases}$$

Accordingly, two types of LMOs are configured in correspondence with two unique values of a two-dimensional array.

The orbital that obtains a stable expected value corresponds to an occupied orbital of a regular molecular orbital whereas the orbital that obtains an unstable expected values corresponds to a virtual orbital. A hybrid orbital that is not used for configuring the LMO of the bond between the atoms is set as the lone electron pair LMO.

In setting the charge data after the number of electrons and the number of orbitals constituting the bond order and the number of lone electron pairs are removed, the number of negative electrons is set when the number of electrons is remaining whereas the number of positive electrons is set when the number of orbitals is remaining. In this example, definitions of each of the symbols in the Formulas (4)-(7) are depicted as follows.

$H^A$ $\Rightarrow$ A:ATOM, H: HYBRID ORBITAL VECTOR (HYBRID ORBITAL VECTOR OF ATOM A)

$H_{\mu}^{A}$ $\Rightarrow$ A:ATOM, $\mu$ : ORBITAL, H: HYBRID ORBITAL VECTOR (ELEMENT PERTAINING TO ORBITAL $\mu$ OF HYBRID ORBITAL VECTOR OF ATOM A)

$\lambda_{+}$ $\Rightarrow$ AMONG THE SOLUTIONS $\lambda$ OF MATRIX FORMULAS, SOLUTION HAVING A POSITIVE SIGN IN FRONT OF SQUARE RO

$\lambda_{-}$ $\Rightarrow$ AMONG THE SOLUTIONS $\lambda$ OF MATRIX FORMULAS, SOLUTION HAVING A NEGATIVE SIGN IN FRONT OF SQUARE RO

$LMO_{AB}^{Occupied}$ $\Rightarrow$ A,B: ATOM, LMO: EITHER $\sigma$ OR $\pi$ (OCCUPIED ORBITAL VECTOR AMONG LMO OF $\sigma$ OR $\pi$ of ATOM A AND B)

$LMO_{AB}^{Virtual}$ $\Rightarrow$ A,B: ATOM, LMO: EITHER $\sigma$ OR $\pi$ (NON-OCCUPIED ORBITAL VECTOR AMONG LMO OF $\sigma$ OR $\pi$ of ATOM A AND B)

$LMO_{A}^{LonePair}$ $\Rightarrow$ A:ATOM, LMO : LONE ELECTRON PAIR (LONE ELECTRON PAIR VECTOR OF ATOM A)

In the case of ethylene, twelve localized molecular orbitals (LMO) are configured as depicted as follows.

$$L1 = \sigma_{C1C2}^{Occupied} = B[1] \to ls[1] \quad , \quad L7 = \sigma_{C2H5}^{Occupied} = B[4] \to ls[1] \quad ,$$

$$L2 = \sigma_{C1C2}^{Virtual} = B[1] \to ls[2] \quad , \quad L8 = \sigma_{C2H5}^{Virtual} = B[4] \to ls[2] \quad ,$$

$$L3 = \sigma_{C1H3}^{Occupied} = B[2] \to ls[1] \quad , \quad L9 = \sigma_{C2H6}^{Occupied} = B[5] \to ls[1] \quad ,$$

$$L4 = \sigma_{C1H3}^{Virtual} = B[2] \to ls[2] \quad , \quad L10 = \sigma_{C2H6}^{Virtual} = B[6] \to ls[2] \quad ,$$

$$L5 = \sigma_{C1H4}^{Occupied} = \text{B[3]} \rightarrow \text{ls[1]} \quad , \qquad L11 = \pi_{C1C2}^{Occupied} = \text{B[1]} \rightarrow \text{lp[1]} \quad ,$$

$$L6 = \sigma_{C1H4}^{Virtual} = \text{B[3]} \rightarrow \text{ls[2]} \quad , \qquad L12 = \pi_{C1C2}^{Virtual} = \text{B[1]} \rightarrow \text{lp[2]}$$

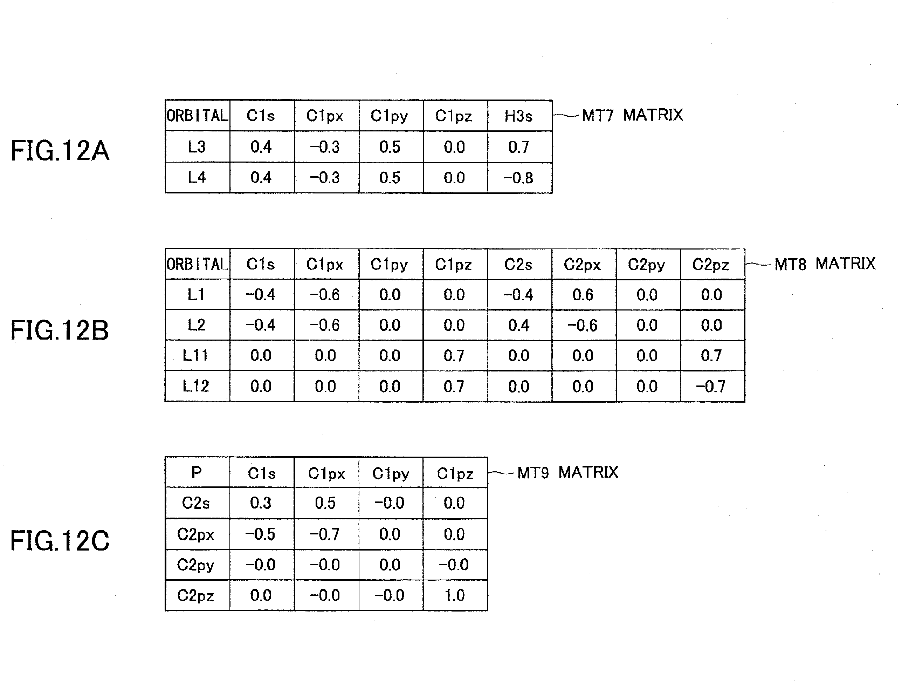

Each of the LMOs depicted above indicates that the LMO is included in the σ orbital data of the bond axis data structure B "B->ls" and the π orbital data of the bond axis data structure B "B->lp". In this example, the LMOs pertaining to the σ bond and the π bond between the atom C1 and the atom H3 correspond to L3 and L4 and are configured in a manner illustrated in L3 and L4 of a matrix MT7 of Fig. 12A. Further, in this example, the LMOs pertaining to the σ bond and the π bond between the atom C1 and the atom C2 correspond to L1, L2 (σ) and L11, L12 (π) and are configured in a manner illustrated in L1, L2 (σ) and L11, L12 (π) of a matrix MT8 of Fig. 12B.

The initial electron density P of ethylene can be calculated with the following Formula (8).

[Formula (8)]

$$P_{\mu\nu} = 2 \sum_{i \in Occupied} Li_{\mu} Li_{\nu}$$

$\Rightarrow$ $\quad$ B[1] $\rightarrow$ pb

(TO DATA STRUCTURE)

The calculation results with Formula (8) indicates that the initial electron density P is included in the density matrix element of the bond axis data structure B "B[1]->pb". For example, the initial electron density P pertaining to the bond between the atom C1 and the atom C2 is configured as illustrated in a matrix MT9 of Fig. 12C in accordance with the calculation using Formula (8).

(Second example)

The process (method) according to the above-described embodiment of the present invention is applied to P1CBN described in Protein Databank (www.pdb.org.) in which a molecule added with a hydrogen atom (Crambin, C202H314N55064S6) is used.

An LMO is configured substantially in the same manner as the first example in which the molecular structure design program 21 is executed in accordance with the flow illustrated in Figs. 5-7.

A value of the initial electron density P calculated by Formula (8) is used as the initial electron density matrix used for the below-described molecular orbital calculation program. It is, however, to be noted that the value of the matrix MT1 of Fig. 9 is only applied to the elements of the same atoms having the same orbital. Because the molecule in this example has electrons of an odd number, two types of densities (density pertaining to up-spin electrodes and density pertaining to down-spin electrodes) are set.

A comparative calculation test is performed where MO-G (manufactured by Fujitsu, software.fujitsu.com/jp/scigress) is used as the molecular orbital calculation program and Q6700 (manufactured by Intel) is used as the CPU of the calculator used for calculation. As a result, the calculated heat of formation by using the initial electron density set by a conventional method is 1993.48 kcal/mol whereas the calculated heat of formation by using the initial electron density set by the process (method) according to the above-described embodiment of the present invention is 1991.10 kcal/mol. Thus, a stable state exhibiting an improvement of approximately 2.4 kcal/mol can be obtained.

The reason that the process (method) according to the above-described embodiment of the present invention can achieve such improved calculation precision is because, unlike the initial electron density of the conventional method, the process (method) according to the above-described embodiment of the present invention can an off-diagonal element of an electron density matrix can be included.

(Third example)

The process (method) according to the above-described embodiment of the present invention is applied to P1CBN described in Protein Databank (www.pdb.org.) in which an anion molecule added with a hydrogen atom (Crambin (-), C202H314N55O64S6) is used.

An LMO is configured substantially in the same manner as the first example in which the molecular structure design program 21 is executed in accordance with the flow illustrated in Figs. 5-7.

A value of the initial electron density P calculated by Formula (8) is used as the initial electron density matrix used for the below-described molecular orbital calculation program. It is, however, to be noted that the value of the matrix MT1 of Fig. 9 is only applied to the elements of the same atoms having the same orbital.

A comparative calculation test is performed where MO-G (manufactured by Fujitsu, software.fujitsu.com/jp/scigress) is used as the molecular orbital calculation program and Q6700 (manufactured by Intel) is used as the CPU of the calculator used for calculation. As a result, the time for calculating molecular orbital (molecular orbital calculation time) by using the initial electron density set by a conventional method is 1028.22 seconds whereas the molecular orbital calculation time by using the initial electron density set by the process (method) according to the above-described embodiment of the present invention is 255.52 seconds. Thus, high speed calculation exhibiting an increased speed of 4 times can be obtained.

The process (method) according to the above-described embodiment of the present invention can achieve such increased calculation speed owing to mainly the method of constructing the initial electron density. For example, in a case of carbon having 4 atom orbitals (e.g., fullerene C60) where 1 atom combined with 3 adjacent atoms is changed, the conventional method requires to construct all 240 orbitals (hybrid orbitals and localized molecular orbitals) whereas the process (method) according to the above-described embodiment of the present invention needs only to construct 16 orbitals (hybrid orbitals and localized molecular orbitals) pertaining to the 4 atoms involved in the change. This difference results in a significant reduction of calculation cost. Although not proportional to the reduction of calculation cost, it can be understood that the total time of molecular orbital calculation (calculation time until the converging of the constructed initial electron density) is significantly reduced compared to the conventional method.

With the above-described embodiments of the present invention, the setting of an initial electron density matrix enabling high speed molecular orbital calculation for a molecule to which an atom is added can be easily and accurately achieved in a short time.

That is, with the process (method) according to the above-described embodiment of the present invention, a molecular structure can be constructed with a GUI and a localized molecular orbital capable of obtaining an appropriate initial electron density can be configured by providing a data structure as illustrated in Figs. 4A-4C including a part having data items pertaining to an atom (e.g., a hybrid orbital, a lone electron pair, a density matrix element) and another part having data items pertaining to a bond axis (e.g., a $\sigma$ orbital, a $\pi$ orbital, a density matrix element, a 2 electron integration), calculating interaction between the atomic orbital of the atoms constituting a new bond and the atomic orbital in-between the atoms by using an algorithm of the flow illustrated in Figs. 5-7 and the data included in the data structure. The molecular structure construction assisting apparatus 30 according to the above-described embodiment of the present invention is configured to perform the process (method) according to the above-described embodiment of the present invention. Further, the recording medium 20 according to the above-described embodiment of the present invention has the molecular structure design program 21 recorded thereon for causing the computer 10 to perform the process (method) according to the above-described embodiment of the present invention.

Compared to the conventional method of configuring an initial electron density by constructing all LMOs of a molecule or the conventional method of using a diagonal matrix for an initial electron density matrix, the use of the initial electron matrix configured by the process (method) according to the above-described embodiment of the present invention significantly reduces calculation cost.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiments of the present invention have been described in detail, it should be understood that the various changes, substitutions, and alterations could be made hereto without departing from the spirit and scope of the invention.

**Claims**

**1.** A molecular structure construction assisting apparatus **characterized by** comprising:

a storage unit configured to store a data structure pertaining to a molecular structure, the data structure including at least

a molecule data structure including an atom data structure pertaining to an atom, a bond axis data structure pertaining to a bond axis, and a molecule main data structure,

an element data structure pertaining to an element of the atom;

a display unit configured to display the molecular structure;

an operation unit configured to instruct a target atom to be arranged in the molecular structure;

a molecular structure design unit configured to derive a hybrid orbital of the target atom and a localized molecular orbital pertaining to a bond formed between the target atom and another atom of the molecular structure by using the data structure.

2. The molecular structure construction assisting apparatus as claimed in claim 1,
   **characterized in that** the storage unit is configured to further store an atom data item in the atom data structure, the atom data item including at least one of an atom sequence number, an element symbol number, a first flag, an atom coordinates, a set number of electrons, an included number of bond axes, an included bond axis number, a hybrid orbital, a number of lone electron pairs, a lone electron pair localized molecular orbital, an atomic charge,
   wherein the storage unit is configured to further store a bond axis data item, the bond axis data item including at least one of a bond axis sequence number, a second flag, a both end atom number, a bond axis vector, a bond order, a σ localized molecular orbital, a π localized molecular orbital, a 2 electron integration,
   wherein the storage unit is configured to further store a molecule main data item in the molecule main data structure, the molecule main data item including at least one of an atom data pointer, a bond axis data pointer, a number of atoms, and a number of all bond axes, and
   wherein the storage unit is configured to further store an element data item in the element data structure, the element data item including at least one of a set reference number of electrons, a set reference number of orbitals, a bond radius, and a reference atom orbital.

3. The molecular structure construction assisting apparatus as claimed in claim 2, **characterized in that** the molecular structure design unit is configured to derive the localized molecular orbital by using at least the σ localized molecular orbital, the π localized molecular orbital, and the lone electron pair localized molecular orbital.

4. The molecular structure construction assisting apparatus as claimed in claim 2, **characterized in that** the molecular structure design unit is configured to double data pertaining to the localized molecular orbital and provide two types of localized molecular orbital data in a case where a total number of electrons of the molecular structure is an odd number.

5. The molecular structure construction assisting apparatus as claimed in one of claims 1-3, **characterized in that** the molecular structure design unit is configured to derive an initial electron density matrix based on the localized molecular orbital.

6. A computer-readable recording medium on which a program is recorded for causing a computer to execute a method for designing a molecular structure, the method **characterized by** comprising:

   storing an atom data pertaining to an atom added to a molecular structure and a bond axis data pertaining to a bond axis connected to the atom in association with a molecular data structure in a storage unit;
   calculating a localized molecular orbital of the bond axis;
   setting the calculated localized molecular orbital as a localized molecular orbital data included in the bond axis data;
   setting atom sequence numbers of atoms provided on both ends of the bond axis in a lone electron pair orbital data included in the atom data; and
   generating an electron density matrix in the storage unit by adding a product of localized molecular orbitals pertaining to an occupied orbital by using the localized molecular orbital data and the lone electron pair orbital data.

7. The computer-readable recording medium as claimed in claim 6, **characterized by** further comprising:

   expanding the molecular structure data in the storage unit based on a data structure including at least a molecule data structure having an atom data structure, a bond axis data structure, and a molecule main data structure, and an element data structure.

8. The computer-readable recording medium as claimed in claim 7, **characterized in that** the expanding includes:

storing an atom data item in the atom data structure, the atom data item including at least one of an atom sequence number, an element symbol number, a first flag, an atom coordinates, a set number of electrons, an included number of bond axes, an included bond axis number, a hybrid orbital, a number of lone electron pairs, a lone electron pair localized molecular orbital, an atomic charge,

storing a bond axis data item, the bond axis data item including at least one of a bond axis sequence number, a second flag, a both end atom number, a bond axis vector, a bond order, a $\sigma$ localized molecular orbital, a $\pi$ localized molecular orbital, a 2 electron integration,

storing a molecule main data item in the molecule main data structure, the molecule main data item including at least one of an atom data pointer, a bond axis data pointer, a number of atoms, and a number of all bond axes, and

storing an element data item in the element data structure, the element data item including at least one of a set reference number of electrons, a set reference number of orbitals, a bond radius, and a reference atom orbital;

wherein the calculating includes calculating the $\sigma$ localized molecular orbital and the $\pi$ localized molecular orbital.

9. The computer-readable recording medium as claimed in one of claims 7 and 8, **characterized in that** the method further comprises:

doubling data pertaining to the localized molecular orbital; and

providing two types of localized molecular orbital data in a case where a total number of electrons of the molecular structure is an odd number.

10. A method for designing a molecular structure, the method **characterized by** comprising:

storing an atom data pertaining to an atom added to a molecular structure and a bond axis data pertaining to a bond axis connected to the atom in association with a molecular data structure in a storage unit;

calculating a localized molecular orbital of the bond axis;

setting the calculated localized molecular orbital as a localized molecular orbital data included in the bond axis data;

setting atom sequence numbers of atoms provided on both ends of the bond axis in a lone electron pair orbital data included in the atom data; and

generating an electron density matrix in the storage unit by adding a product of localized molecular orbitals pertaining to an occupied orbital by using the localized molecular orbital data and the lone electron pair orbital data.

FIG.1

10

EP 2 479 695 A2

| 11 | 12 | 13 | 14 |
|---|---|---|---|
| CPU | MEMORY DEVICE | DISPLAY DEVICE | OUTPUT DEVICE |

SYSTEM BUS — 19

| 15 | 16 | 17 | 18 |
|---|---|---|---|
| OPERATION DEVICE | COMMUNICATION DEVICE | STORAGE DEVICE | DRIVER |

RECORDING MEDIUM

20

# FIG.2

EP 2 479 695 A2

FIG.3

CONSTRUCT
INITIAL DATA STRUCTURE ～S1

3a

H        H

C ＝ C

H            H

ALLOCATE ATOMS WITH GUI ～S2

3b-2        3b ATOMIC ORBITAL

C        H

CALCULATE INTERACTION OF ATOMIC ORBITAL OF ATOM
AND ATOMIC ORBITAL IN-BETWEEN ATOMS ～S3

3c-2        3c HYBRID ORBITAL

C        H

SET HYBRID ATOMIC ORBITAL
BY DIAGONALIZING INTERACTION MATRIX ～S4

3d-2        3d LMO($\sigma$)

C        H

SET LMO BY USING HYBRID ATOMIC ORBITAL
AND FOCK MATRIX ELEMENT ～S5

FIG.4A

EP 2 479 695 A2

~40

| 41 | | OMOLECULE DATA POINTER(M)<br>OELEMENT DATA(EI)<br>OEVENT DATA POINTER(E) | |
|---|---|---|---|
| 42 | M | OATOM DATA POINTER(A)<br>OBOND AXIS DATA POINTER(B)<br>ONUMBER OF ATOMS(n)<br>ONUMBER OF BOND AXES(nb) | }42m |
| | ATOM<br>(M→A) | (ATOM DATA) | }42a |
| | AXIS<br>(M→B) | (BOND AXIS DATA) | }42b |
| 43 | EI | OSET REFERENCE NUMBER OF ELECTRONS(n0)<br>OSET REFERENCE NUMBER OF ORBITALS(m0)<br>OATOMIC ORBITAL(ao)<br>OBOND RADIUS DATA(r) | |
| 44 | E | OPERATION DATA (EVENT DATA) | |

FIG.4B

```
                                           A
┌─────────────────────────────────────────┐
│ ATOM DATA:M→A                             │
├─────────────────────────────────────────┤
│ ○ATOM SEQUENCE NUMBER(a)                  │
│ ○ELEMENT SYMBOL(N)                        │
│ ○COORDINATES(c)                           │
│ ○INCLUDED NUMBER OF BOND AXES(nb)         │
│ ○INCLUDED BOND AXIS NUMBER(b)             │
│ ○EVENT FLAG(f)                            │
│ ●NUMBER OF ELECTRONS(n)                   │
│ ○NUMBER OF ORBITALS(m)                    │
│ ●HYBRID ORBITAL DATA(ho)                  │
│ ●NUMBER OF LONE ELECTRON PAIRS(nl)        │
│ ●LONE ELECTRON PAIR ORBITAL DATA(ll)      │
│ ○ATOMIC CHARGE(q)                         │
│ ●DENSITY MATRIX ELEMENT DATA(pa)          │
│ ○ATOMIC ORBITAL (ao)                      │
└─────────────────────────────────────────┘
```

FIG.4C

```
                                           B
┌─────────────────────────────────────────┐
│ BOND AXIS DATA:M→B                        │
├─────────────────────────────────────────┤
│ ○BOND AXIS SEQUENCE NUMBER(b)             │
│ ○VECTOR DATA(v)                           │
│ ○BOND ORDER(bt)                           │
│ ○BOTH END ATOM NUMBER(a)                  │
│ ○EVENT FLAG(f)                            │
│ ●σ ORBITAL DATA(ls)                       │
│ ●π ORBITAL DATA(lp)                       │
│ ●DENSITY MATRIX ELEMENT DATA(pb)          │
│ ○2ELECTRON INTEGRATION DATA(int2el)       │
│ ○BOND FLAG (bf)                           │
└─────────────────────────────────────────┘
```

FIG.5

```
                    ┌─────────────┐
                    │    Start    │
                    └──────┬──────┘
                           │
              ╱────────────────────────╲
             ╱   Construct M, EI         ╲    ～S11
             ╲                           ╱
              ╲────────────────────────╱
                           │
            ╱──────────────────────────────╲   ～S12
           ╱   Input coordinates of atoms    ╱
          ╱────────────────────────────────╱
                           │
                           ▼
            ┌────────────────────────────┐   ～S13
            │         Set M→n             │
            └─────────────┬──────────────┘
                           │
                           ▼
            ┌────────────────────────────┐   ～S14
            │    Allocate M→A[1:M→n]      │
            └─────────────┬──────────────┘
                           │
              ╱────────────────────────╲    ～S15
             ╱   i = 0, M→nb = 0         ╲
             ╲                           ╱
              ╲────────────────────────╱
                           │
            ┌────────────────────────────┐   ～S16
            │        i = i +1, j = i      │
            └─────────────┬──────────────┘
                           │
                           ▼
            ┌────────────────────────────┐
            │          j = j + 1          │  ─S17
            └─────────────┬──────────────┘
                           │
                           ▼
         ╱─────────────────────────────────╲  ～S18      NO
        ╱  ‖ A[i]→c−A[j]→c ‖ < Σ EI[A[i, j]]→N →r ╲──────────
        ╲                  ?                     ╱
         ╲─────────────────────────────────────╱
                           │ YES
                           ▼
            ┌────────────────────────────┐   ～S19
            │     M→nb = M→nb +1          │
            └─────────────┬──────────────┘
                           │
              NO           ▼
        ◄──────── ╱──────────────────╲   ～S20
                  ╲    j == M→n?      ╱
                   ╲────────────────╱
                           │ YES
              NO           ▼
        ◄──────── ╱──────────────────╲   ～S21
                  ╲   i == M→n − 1?   ╱
                   ╲────────────────╱
                           │ YES
                           ▼                 ～S22
            ┌────────────────────────────┐
            │    Allocate M→B[1:M→nb]     │
            └─────────────┬──────────────┘
                           │
                    ┌──────▼──────┐
                    │     End     │
                    └─────────────┘
```

FIG.6

```
                    ┌──────────────┐
                    │    Start     │
                    └──────┬───────┘
                           │
          ⬡ Construct M, E, El, A, B ⬡         S31
                           │
         ⬡ Input coordinates of N atoms ⬡      S32
                           │
   ┌──────────────────────────────────────┐
   │ Set A[M→n+1:M→n+N], M→n= M→n+ N        │  S33
   └──────────────────┬─────────────────────┘
                       │
   ⬡ i = 0, j = M→n − N, k = M→nb, l = 0 ⬡    S34
```

Set $A[M{\to}n+1:M{\to}n+N]$, $M{\to}n = M{\to}n+ N$ — S33

$i = 0$, $j = M{\to}n - N$, $k = M{\to}nb$, $l = 0$ — S34

S35: $j = j + 1$

S36: $i = i + 1$

S37: $\| A[i]{\to}c - A[j]{\to}c \| < \sum El[A[i, j]{\to}N]{\to}r$ ?  —  NO

YES

S38: $M{\to}nb = M{\to}nb + 1$, $A[i, j]{\to}nb = A[i, j]{\to}nb + 1$

S39: $A[i, j]{\to}m(\text{or } n) = A[i, j]{\to}m(\text{or } n) - A[i, j]{\to}nb$

S40: $k = k + 1$, $l = l + 1$

S41: Set $B[k]$ ($B[k]{\to}bt = 1$), $A[i]{\to}b \ni k$, $A[j]{\to}b \ni k$

S42: Set $B[k]{\to}int2el$, $A[i, j]{\to}ho$
(Calculate Fock elements for atom i and j)

S43: $i == j$?  — NO

YES

S44: $j == M{\to}n$?  — NO

YES

( 1 )

FIG.7

Set B→bt, A→q, A→nl, A→m, A→n, A→bf — S45

7a

i = M→nb − 1 — S46

i = i + 1 — S47

Set Sigma LMO B[i]→ls — S48

j = 0 — S49

j = j + 1 — S50

Set Pi LMO B[i]→lp — S51

NO ← j == B[i]→bt − 1 — S52

YES

j = 0 — S53

j = j + 1 — S54

Set Lone Pair LMO A[B[i]→a[1,2]]→ll — S55

NO ← j == A[B[i]→a[1,2]]→nl — S56

YES

NO ← i == M→nb — S57

YES

End

# FIG.8A

# FIG.8B

8a : C-C : 1.3 Å
8b : C-H : 1.0 Å
8c : ∠HCH : 120°

# FIG.9

MT1 MATRIX

$P_{\mu\nu}$

| ORBITAL | H3s |
|---|---|
| H3s | 1.0 |

| ORBITAL | H4s |
|---|---|
| H3s | 0.0 |

| ORBITAL | C1s | C1px | C1py | C1pz |
|---|---|---|---|---|
| H3s | 0.0 | 0.0 | 0.0 | 0.0 |

| ORBITAL | C1s | C1px | C1py | C1pz |
|---|---|---|---|---|
| C1s | 1.0 | 0.0 | 0.0 | 0.0 |
| C1px | 0.0 | 1.0 | 0.0 | 0.0 |
| C1py | 0.0 | 0.0 | 1.0 | 0.0 |
| C1pz | 0.0 | 0.0 | 0.0 | 1.0 |

| ORBITAL | C1s | C1px | C1py | C1pz |
|---|---|---|---|---|
| C2s | 0.0 | 0.0 | 0.0 | 0.0 |
| C2px | 0.0 | 0.0 | 0.0 | 0.0 |
| C2py | 0.0 | 0.0 | 0.0 | 0.0 |
| C2pz | 0.0 | 0.0 | 0.0 | 0.0 |

# FIG.10

MT2 MATRIX

| ORBITAL | C1s | C1px | C1py | C1pz | C2s | H3s | H4s |
|---------|-----|------|------|------|-----|-----|-----|
| C1s | 0.0 | 0.0 | 0.0 | 0.0 | −7.4 | −6.6 | −6.6 |
| C1px | 0.0 | 0.0 | 0.0 | 0.0 | −5.4 | 1.8 | 1.8 |
| C1py | 0.0 | 0.0 | 0.0 | 0.0 | 0.0 | −3.1 | 3.1 |
| C1pz | 0.0 | 0.0 | 0.0 | 0.0 | 0.0 | 0.0 | 0.0 |
| C2s | −7.4 | −5.4 | 0.0 | 0.0 | 0.0 | 0.0 | 0.0 |
| H3s | −6.6 | 1.8 | −3.1 | 0.0 | 0.0 | 0.0 | 0.0 |
| H4s | −6.6 | 1.8 | 3.1 | 0.0 | 0.0 | 0.0 | 0.0 |

FIG.11A

MATRIX MT3

| ORBITAL | C1s | C1px | C1py | C1pz | C2s | H3s | H4s |
|---|---|---|---|---|---|---|---|
| C1s | 0.0 | 0.0 | 0.0 | 0.0 | −7.4 | −6.6 | −6.6 |
| C1px | 0.0 | 0.0 | 0.0 | 0.0 | −5.4 | 1.8 | 1.8 |
| C1py | 0.0 | 0.0 | 0.0 | 0.0 | 0.0 | −3.1 | 3.1 |
| C1pz | 0.0 | 0.0 | 0.0 | 0.0 | 0.0 | 0.0 | 0.0 |
| C2s | −7.4 | −5.4 | 0.0 | 0.0 | 0.0 | 0.0 | 0.0 |
| H3s | −6.6 | 1.8 | −3.1 | 0.0 | 0.0 | 0.0 | 0.0 |
| H4s | −6.6 | 1.8 | 3.1 | 0.0 | 0.0 | 0.0 | 0.0 |

FIG.11B

MATRIX MT4

| ORBITAL | C1s | C1px | C1py | C1pz | C2s | H3s | H4s |
|---|---|---|---|---|---|---|---|
| 1 | 0.7 | 0.1 | −0.0 | −0.0 | 0.5 | 0.4 | 0.4 |
| 2 | 0.1 | −0.7 | −0.0 | −0.0 | −5.0 | 0.3 | 0.3 |
| 3 | 0.0 | −0.0 | 0.7 | 0.0 | −0.0 | 0.5 | −0.5 |
| 4 | 0.0 | −0.0 | −0.0 | 1.0 | 0.0 | 0.0 | 0.0 |

FIG.11C

MATRIX MT5

| ORBITAL | C1s | C1px | C1py | C1pz | C2s | H3s | H4s |
|---|---|---|---|---|---|---|---|
| H$^{C1}$1 | 0.4 | −0.3 | 0.5 | −0.0 | 0.0 | 0.7 | 0.0 |
| H$^{C1}$2 | −0.4 | −0.6 | 0.0 | 0.0 | −0.7 | 0.0 | 0.0 |
| H$^{C1}$3 | −0.4 | 0.3 | 0.5 | 0.0 | 0.0 | 0.0 | −0.7 |
| H$^{C1}$4 | 0.0 | −0.0 | −0.0 | 1.0 | 0.0 | 0.0 | 0.0 |

FIG.11D

MATRIX MT6

| ORBITAL | C1s | C1px | C1py | C1pz |
|---|---|---|---|---|
| H$^{C1}$1 | 0.6 | −0.4 | 0.7 | −0.0 |
| H$^{C1}$2 | −0.6 | −0.8 | 0.0 | 0.0 |
| H$^{C1}$3 | −0.6 | 0.4 | 0.7 | 0.0 |
| H$^{C1}$4 | 0.0 | −0.0 | −0.0 | 1.0 |

A[1] → ho
(TO DATA STRUCTURE)

EP 2 479 695 A2

## FIG.12A

MT7 MATRIX

| ORBITAL | C1s | C1px | C1py | C1pz | H3s |
|---|---|---|---|---|---|
| L3 | 0.4 | -0.3 | 0.5 | 0.0 | 0.7 |
| L4 | 0.4 | -0.3 | 0.5 | 0.0 | -0.8 |

## FIG.12B

MT8 MATRIX

| ORBITAL | C1s | C1px | C1py | C1pz | C2s | C2px | C2py | C2pz |
|---|---|---|---|---|---|---|---|---|
| L1 | -0.4 | -0.6 | 0.0 | 0.0 | -0.4 | 0.6 | 0.0 | 0.0 |
| L2 | -0.4 | -0.6 | 0.0 | 0.0 | 0.4 | -0.6 | 0.0 | 0.0 |
| L11 | 0.0 | 0.0 | 0.0 | 0.7 | 0.0 | 0.0 | 0.0 | 0.7 |
| L12 | 0.0 | 0.0 | 0.0 | 0.7 | 0.0 | 0.0 | 0.0 | -0.7 |

## FIG.12C

MT9 MATRIX

| P | C1s | C1px | C1py | C1pz |
|---|---|---|---|---|
| C2s | 0.3 | 0.5 | -0.0 | 0.0 |
| C2px | -0.5 | -0.7 | 0.0 | 0.0 |
| C2py | -0.0 | -0.0 | 0.0 | -0.0 |
| C2pz | 0.0 | -0.0 | -0.0 | 1.0 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7168807 A
- JP 2003012567 A

- JP 2001319179 A

**Non-patent literature cited in the description**

- **P.W. ATKINS ; HIDEAKI CHIHARA ; NOBUO NA-KAMURA.** Atkins Physical Chemistry. Kagaku Dojin, February 2001, vol. 1, 2